(19) Europäisches Patentamt
European Patent Office
Office européen des brevets

(11) EP 0 611 207 B1

(12) **FASCICULE DE BREVET EUROPEEN**

(45) Date de publication et mention de la délivrance du brevet:
**20.06.2001 Bulletin 2001/25**

(51) Int Cl.[7]: **A61K 9/127**, A61K 7/00, A61K 47/36

(21) Numéro de dépôt: **94400271.6**

(22) Date de dépôt: **09.02.1994**

(54) **Procédé de stabilisation de vésicules de lipide(s) amphiphile(s) et composition pour application topique contenant lesdites vésicules stabilisées**

Verfahren zur Stabilisierung von Vesikeln aus amphiphilen Lipiden und diese stabilisierte Vesikeln enthaltende Zusammensetzung für die topische Anwendung

Process for stabilisation of vesicles of amphiphilic lipids and composition for topical application containing these stabilized vesicles

(84) Etats contractants désignés:
**AT BE CH DE DK ES FR GB GR IE IT LI NL PT SE**

(30) Priorité: **12.02.1993 FR 9301612**

(43) Date de publication de la demande:
**17.08.1994 Bulletin 1994/33**

(73) Titulaire: **L'OREAL**
**75008 Paris (FR)**

(72) Inventeurs:
- **Ribier, Alain**
  **F-75001 Paris (FR)**
- **Simonnet, Jean-Thierry**
  **F-75011 Paris (FR)**

(74) Mandataire: **Peuscet, Jacques et al**
**SCP Cabinet Peuscet et Autres,**
**78, avenue Raymond Poincaré**
**75116 Paris (FR)**

(56) Documents cités:
**EP-A- 0 043 327**
**EP-A- 0 437 368**
**FR-A- 2 532 191**
**FR-A- 2 622 104**

- **PATENT ABSTRACTS OF JAPAN vol. 13, no. 208 (C-596) 16 Mai 1989 & JP-A-01 027 634 (SAN EI CHEM IND LTD) 30 Janvier 1989**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne un procédé de stabilisation de vésicules de lipide(s) amphiphile(s) et une composition pour application topique contenant lesdites vésicules stabilisées.

Il est bien connu que certains lipides amphiphiles sont susceptibles de former des vésicules en présence d'eau. De façon connue, les vésicules sont constituées d'une membrane formée d'un ou plusieurs feuillets lipidiques encapsulant une phase généralement aqueuse. La membrane lipidique est préparée à partir d'une phase lipidique (dite vésiculaire) contenant au moins un lipide amphiphile ionique et/ou non-ionique et, généralement des additifs de caractère lipidique susceptibles d'améliorer la stabilité des vésicules et/ou de diminuer leur perméabilité ; la phase lipidique vésiculaire peut également contenir des actifs cosmétiques et/ou pharmaceutiques qui sont lipophiles. La phase aqueuse encapsulée peut contenir également des actifs cosmétiques et/ou pharmaceutiques hydrophiles. Les vésicules de lipide amphiphile sont généralement préparées et utilisées sous forme de dispersion dans une phase aqueuse de dispersion. La phase aqueuse de dispersion peut également contenir des actifs hydrophiles.

Les vésicules de lipide(s) amphiphile(s) et leurs procédés de préparation sont décrits dans de nombreux documents, en particulier dans "Les liposomes. Applications thérapeutiques" Technique et documentation - Lavoisier 1985 et dans "Liposomes en biologie cellulaire et pharmacologie", Edition INSERM. John Libbey Emotext 1987.

Il a été proposé d'utiliser les dispersions de vésicules de lipide(s) amphiphile(s) pour la fabrication de compositions cosmétiques et/ou pharmaceutiques. Ces compositions contiennent généralement des agents tensioactifs, plus particulièrement lorsqu'elles se présentent sous forme de crème ou de pommade ; en effet, dans ce cas, elles comportent une phase grasse qui est généralement émulsionnée à l'aide d'un agent tensioactif.

Malheureusement, les vésicules de lipide(s) amphiphile(s) ont une faible stabilité en présence d'agents tensioactifs et plus particulièrement en présence d'un agent tensioactif et d'une phase grasse. En effet, les agents tensioactifs investissent la membrane de phase lipidique des vésicules jusqu'à ce que lesdites vésicules soient transformées en micelles mixtes. Ce phénomène d'instabilité des vésicules croît avec le temps et la température. Ainsi, dans des compositions formulées sous forme d'émulsion d'une phase grasse, les vésicules de lipide(s) amphiphile(s) incorporées peuvent être détruites après un temps de stockage relativement faible, avant même toute utilisation. De nombreuses études réalisées en microscopie électronique ont en effet montré qu'après un certain temps de conservation la présence de vésicules ne pouvaient plus être decelée.

La demanderesse a trouvé, comme décrit par exemple dans FR-A 2 490 504, que les vésicules de lipide(s) amphiphile(s) sont susceptibles de maintenir sous forme de dispersion stable dans une phase aqueuse des huiles et, plus généralement, des liquides non-miscibles à l'eau, sans qu'il soit nécessaire d'ajouter des agents émulsionnants. On obtient ainsi des compositions cosmétiques dans lesquelles les vésicules sont généralement suffisamment stables. Mais dans ces compositions, les vésicules et la phase huileuse se séparent rapidement lors de l'application sur la peau et, par conséquent, la texture de ces compositions est différente de celle d'une émulsion. L'appréciation sensorielle des utilisatrices est donc différente et une fraction importante de celles-ci reste attachée à la texture classique des émulsions.

Par ailleurs, de nombreux auteurs ont préconisé de stabiliser les vésicules dans des matrices rigides de gel aqueux. Dans ce but, on a proposé l'utilisation de différents agents gélifiants : des polysaccharides, des polypeptides, de la gélatine ou de l'agarose à raison de 0,5 à 10 % en poids de la phase aqueuse de dispersion du gel selon EP-A 0 162 724 ; de la gomme arabique, de l'alginate de sodium, du xanthane, du collagène, des polyacrylates ou de la gélatine selon EP-A 0 172 907 ; de l'ADN à poids moléculaire élevé à raison de 0,1 à 10 % en poids selon FR-A 2 668 063. Mais ces procédés permettent uniquement de préparer des compositions sous forme de gels aqueux rigides, qui sont mal appréciés sur le plan cosmétique.

Selon WO-A 87 01587, pour stabiliser les vésicules de lipide(s) amphiphile(s), on les séquestre dans des microcapsules constituées d'un sel d'alginate et de gélatine. On obtient ainsi des microcapsules, qui peuvent être mises en suspension dans une phase aqueuse continue ou une phase huileuse contenant au moins un agent émulsionnant. Ce procédé de stabilisation des vésicules de lipide(s) amphiphile(s) est compliqué et, par conséquent, coûteux. De plus, les propriétés des vésicules de lipide(s) amphiphile(s) sont masquées par la couche de microencapsulation.

Dans FR-A 2 622 104 et 2 645 455, est décrit un procédé de stabilisation dans lequel on mélange la dispersion de vésicules avec une solution mixte d'un collagène particulier : l'atélocollagène, et de polysaccharides particuliers : les glycosaminoglycannes. Cette solution mixte serait susceptible de protéger les vésicules de lipide(s) amphiphile(s) sans qu'il y ait formation d'un gel rigide mais, en quelque sorte, enrobage des vésicules, ce qui permettrait leur incorporation ultérieure dans des formulations cosmétiques contenant une phase grasse, en particulier des émulsions. Mais cette protection nécessite l'utilisation de proportions relatives élevées en agent(s) stabilisant(s) par rapport au(x) lipide(s) vésiculaire(s) : selon l'exemple 1, on a 0,5 % d'agent stabilisant pour 1 % en poids de lipide, soit 50 % d'agent stabilisant par rapport à la phase lipidique. Ce procédé présente donc l'inconvénient de modifier profondément les caractéristiques propres aux vésicules de lipide(s), qui se trouvent ainsi masquées par des quantités importantes

d'agent(s) stabilisant(s). En effet, on a observé au microscope optique que les vésicules se présentent sous l'aspect d'amas ayant perdu leur individualité propre. On a également constaté que l'adjonction de ce type d'agents stabilisants n'est pas dénuée d'antigénicité.

De plus, on a constaté que l'adjonction de la plupart des agents stabilisants connus n'a pas un effet protecteur suffisant lorsque les vésicules de lipide(s) amphiphile(s) sont en présence des principaux agents tensioactifs couramment utilisés en cosmétique.

Selon la présente invention, on a trouvé que quatre types d'agents gélifiants, qui n'avaient pas été jusqu'à présent proposés pour la stabilisation des vésicules de lipide(s) amphiphile(s) pouvaient être utilisés en proportion suffisamment faible par rapport à la phase lipidique pour ne pas modifier les propriétés intrinsèques des vésicules et que, de plus, ils permettaient de stabiliser lesdites vésicules même en présence d'agent(s) tensioactif(s). Ces agents gélifiants sont les alginates de glycérol, les alginates de propylèneglycol, la gomme de gélane et la gomme de wélane.

La présente invention a donc pour objet un procédé de stabilisation de vésicules formées d'une membrane de phase lipidique contenant au moins un lipide amphiphile ionique et/ou non-ionique encapsulant une phase aqueuse, sous forme de dispersion dans une phase aqueuse, par addition dans la phase aqueuse de dispersion d'au moins un agent stabilisant, caractérisé par le fait que le ou les agent(s) stabilisant(s) est (sont) choisi(s) dans le groupe formé par les alginates de glycérol, les alginates de propylèneglycol, la gomme de gélane et la gomme de wélane.

L'alginate de propylèneglycol a la formule (I) ci-après :

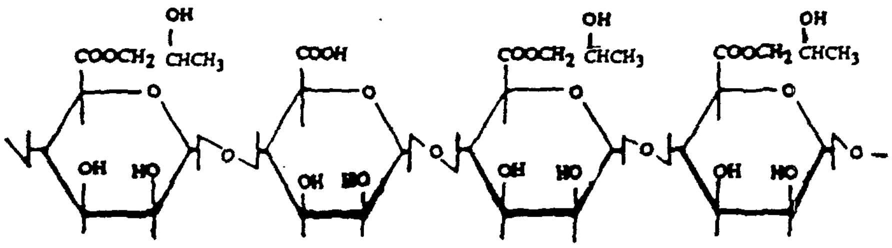

(I)

On utilise, de préférence, des alginates estérifiés à plus de 60 % par du propylèneglycol. Les alginates à plus faible taux d'estérification ont une action stabilisante nettement plus faible. Les alginates de propylèneglycol estérifiés à 80-85 %, qui sont commercialisés sous les dénominations commerciales "KELCOLOID O" ou "MANUCOL ESTER E/PL"par la Société "KELCO", conviennent parfaitement.

L'alginate de glycérol a la formule (I') ci-après :

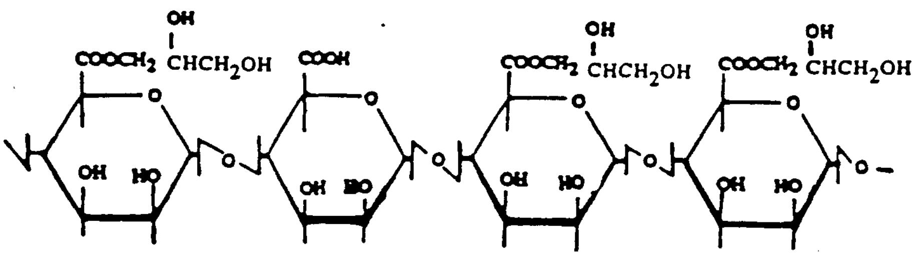

(I')

On utilise, de préférence, des alginates de glycérol estérifiés à plus de 60 % par du glycérol.

La gomme de gélane a une structure tétrasaccharidique récurrente formée d'unités (glucose-acide glucuronique-glucose-rhamnose) et correspond à la formule II suivante

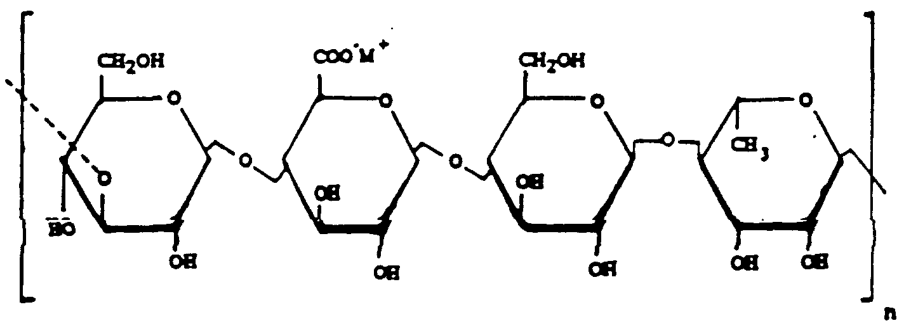

(II)

La gomme de gélane est généralement préparée par fermentation aérobie submergée de Pseudomonas elodea. Une gomme de ce type est commercialisée par la société "KELCO" sous la dénomination commerciale "KELCOGEL" et convient parfaitement. La gomme de gélane purifiée vendue sous la dénomination commerciale "KELCOGEL PC (Personal Care)" par la société "KELCO" convient également.

La gomme de wélane est une gomme de gélane modifiée. Elle a une structure pentasaccharidique récurrente formée d'unités [glucose-acide glucuronique-glucose (rhamnose ou mannose)-rhamnose] et correspond à la formule III suivante

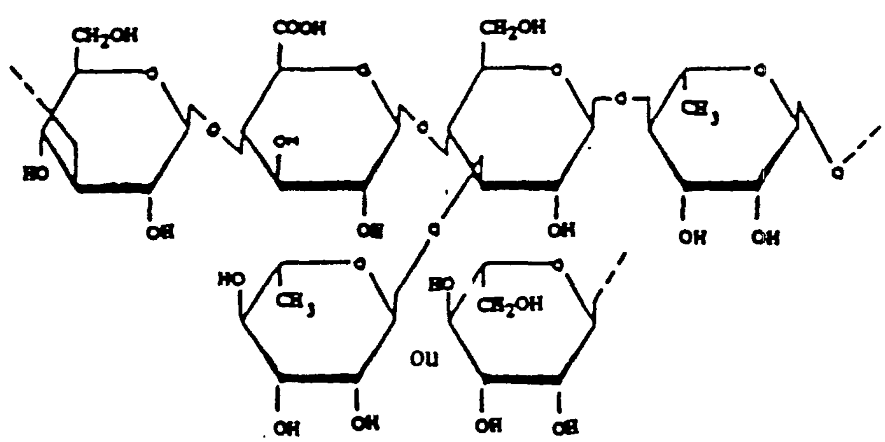

(III)

Elle est préparée, en général, par fermentation de Alcaligenes souche ATCC 31 555. Une gomme de ce type est commercialisée par la société "KELCO" sous la dénomination commerciale "K 1 A 96" et convient parfaitement.

Les agents stabilisants selon l'invention sont utilisés à raison de 0,1 à 20 % en poids par rapport au poids de la phase lipidique vésiculaire. Pour des quantités supérieures à 20%, les propriétés intrinsèques des vésicules risquent d'être modifiées et le gel de devenir rigide et pour des rapports inférieurs à 0,1 % on n'observe plus suffisamment d'effet protecteur. Le rapport préféré selon l'invention est compris entre 0,2 et 10 % ; il est plus particulièrement voisin de 2 % en poids d'agent(s) stabilisant(s) par rapport au poids de la phase lipidique vésiculaire.

Les agents stabilisants ci-dessus sont couramment utilisés dans l'alimentation humaine et ont l'avantage de ne pas présenter d'antigénicité.

Le procédé de stabilisation ci-dessus a l'avantage de permettre la stabilisation des vésicules même dans une composition contenant un agent tensioactif et/ou une phase grasse. On a constaté qu'une composition aqueuse renfermant une dispersion aqueuse de vésicules contenant jusqu'à 20 % en poids d'agent tensioactif par rapport au poids total de la composition était stable. De plus les vésicules restent stables, lorsque la phase grasse associée à la phase aqueuse de dispersion représente jusqu'à 50 % en poids par rapport au poids total de la composition, en présence d'agents tensioactifs autres que les lipides constitutifs des vésicules.

Lorsque les compositions contiennent une phase grasse, celle-ci peut être toute phase grasse généralement utilisée pour la préparation de compositions cosmétiques ou pharmaceutiques pour application topique, sous forme de pommade, crème, lotion ou lait. L'agent tensioactif peut être tout agent tensioactif utilisé pour la préparation de compositions cosmétiques ou pharmaceutiques sous forme d'émulsion d'une phase grasse de type eau dans l'huile ou huile dans l'eau. Une stabilisation convenable peut être obtenue avec les quantités d'agent tensioactif et/ou de phase grasse généralement utilisées pour la fabrication de compositions cosmétiques ou pharmaceutiques pour application topique.

Selon l'invention, il est possible de stabiliser toutes les vésicules de phase lipidique connues, qu'elles soient préparées à partir de lipide(s) amphiphile(s) ionique(s) et/ou non-ionique(s).

La présente invention concerne aussi une composition contenant une dispersion de vésicules de phase lipidique stabilisées par le procédé décrit ci-dessus.

La présente invention a donc également pour objet une composition pour application topique, notamment cosmétique ou pharmaceutique, contenant, en premier lieu, une dispersion de vésicules formées d'une membrane de phase lipidique contenant au moins un lipide amphiphile ionique et/ou non-ionique encapsulant une phase aqueuse, dispersées dans une phase aqueuse, et, en second lieu, au moins un agent stabilisant desdites vésicules, caractérisée par le fait que l'(les) agent(s) stabilisant(s) est(sont) choisi(s) dans le groupe formé par les alginates de glycérol, les alginates de propylèneglycol, la gomme de gélane et la gomme de wélane.

Dans la composition selon l'invention, les alginates sont, de préférence, des alginates estérifiés à au moins 60 % par du propylèneglycol ou du glycérol.

L'(les) agent(s) stabilisant(s) représente(nt) de 0,1 à 20 % en poids par rapport au poids de la phase lipidique vésiculaire, de préférence de 0,2 à 10 %.

Les compositions selon l'invention peuvent contenir jusqu'à 20 % en poids d'agent(s) tensioactif(s) par rapport au poids total de la composition. L'(les) agent(s) tensioactif(s) représente(nt), de préférence, de 1 à 20 % en poids par rapport au poids total de la composition.

Les agents tensioactifs utilisables peuvent être, de façon connue, choisis parmi les composés amphiphiles non-ioniques, anioniques, cationiques ou amphotères tels que, par exemple, les alkylsulfates de sodium, les esters de polyols oxyéthylénés ou non tels que, par exemple, les esters de glycérol, de sorbitol et d'anhydride de sorbitol ; les alcools polyoxyéthylénés ; les alcools polypropylénés ; les copolymères polyoxyéthylène/polyoxypropylène. A titre d'alkylsulfate de sodium, on peut citer le lauryl sulfate de sodium. A titre d'esters de polyols, on peut citer les stéarates de polyoxyéthylène, tels que les produits vendus sous les dénominations commerciales "MYRJ 52" ou "MYRJ 53" par la société "ICI", ainsi que les mélanges de stéarate de sorbitol oxyéthylénés tels que les produits vendus sous les dénominations commerciales "TWEEN 20" et "TWEEN 60" par la société "ICI". A titre d'alcool polyoxyéthyléné, on peut citer l'éther de polyéthylèneglycol de l'alcool stéarylique tel que le produit vendu sous la dénomination commerciale "BRIJ 72" par la société "ICI". A titre d'alcool polypropyléné, on peut citer l'éther de polypropylèneglycol de l'alcool stéarylique tel que le produit vendu sous la dénomination commerciale "ARLAMOL E" par la société "ICI". A titre de copolymère de polyoxyéthylène/ polyoxypropylène, on peut citer le produit vendu sous la dénomination commerciale "POLOXAMER 188" par la société "ICI". La dispersion de vésicules de phase lipidique utilisée pour la préparation de la composition selon l'invention peut être toute dispersion de vésicules connue.

La phase lipidique constitutive des membranes des vésicules de la dispersion peut donc comprendre, de façon connue, au moins un lipide choisi dans le groupe formé par :

A) les lipides non-ioniques ci-après définis :

(1) les dérivés du glycérol, linéaires ou ramifiés, de formule

$$R_oO-\left[C_3H_5(OH)O\right]_{\bar{n}}-H \quad \text{(IV)}$$

formule (IV) dans laquelle :

- $-C_3H_5(OH)O-$ est représenté par les structures suivantes prises en mélange ou séparément : $-CH_2CHOHCH_2O-$ ,

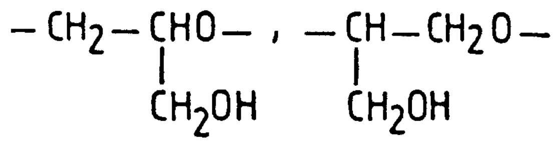

- n est une valeur statistique moyenne comprise entre 1 et 6 ou bien n = 1 ou 2 et alors $-C_3H_5(OH)O-$ est représenté par la structure $-CH_2CHOH-CH_2O-$ ;

- $R_o$ représente :

(a) une chaîne aliphatique, linéaire ou ramifiée saturée ou insaturée, contenant de 12 à 30 atomes de carbone ; ou des radicaux hydrocarbonés des alcools de lanoline ; ou les restes d'alpha-diols à longue chaîne ;
(b) un reste $R_1CO$, où $R_1$ est un radical aliphatique, linéaire ou ramifié, en $C_{11}$-$C_{29}$;
(c) un reste

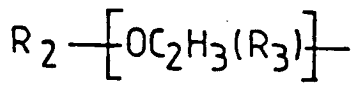

où :

- . $R_2$ peut prendre la signification (a) ou (b) donnée pour $R_o$ ;
- . $-OC_2H_3(R_3)-$
est représenté par les structures suivantes, prises en mélange ou séparément :

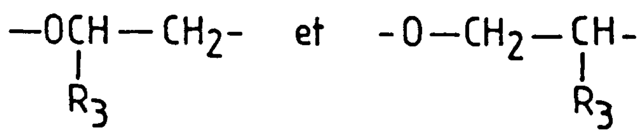

où $R_3$ prend la signification (a) donnée pour $R_0$ ;

(2) les éthers de polyglycérol, linéaires ou ramifiés, comportant deux chaînes grasses ;
(3) les diols à chaîne grasse ;
(4) les alcools gras oxyéthylénés ou non, les stérols tels par exemple le cholestérol et les phytostérols, oxyéthylénés ou non ;
(5) les éthers et esters de polyols, oxyéthylénés ou non, l'enchaînement des oxydes d'éthylène pouvant être linéaire ou cyclique ; par esters de polyols, on entend notamment les esters d'au moins un polyol choisi dans le groupe formé par les oxydes d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités glycérol, les sucroses, les glucoses portant 2 à 30 unités d'oxyde d'éthylène et d'au moins un acide gras comportant une chaîne alkyle en $C_5$-$C_{17}$, saturée ou non saturée, linéaire ou ramifiée, le nombre de chaînes alkyle par groupe polyol étant compris entre 1 et 10 ;
(6) les glycolipides d'origine naturelle ou synthétique, les éthers et esters de mono ou polysaccharides et notamment les éthers et les esters de glucose ;
(7) les hydroxyamides décrits dans le brevet français n° 2 588 256 et représentés par la formule :

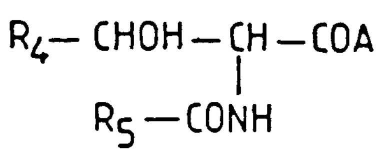 (V)

formule (V) dans laquelle :

- $R_4$ désigne un radical alkyle ou alcényle en $C_7$-$C_{21}$ ;
- $R_5$ désigne un radical hydrocarboné, saturé ou insaturé, en $C_7$-$C_{31}$ ;
- COA désigne un groupement choisi parmi les deux groupements suivants :

  . un reste

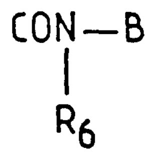

où :

- . B est un radical alcoyle dérivé d'amines primaires ou secondaires, mono- ou polyhydroxylées ; et
- . $R_6$ désigne un atome d'hydrogène ou un radical méthyle, éthyle ou hydroxyéthyle ; et
- . un reste -COOZ, où Z représente le reste d'un polyol en $C_3$-$C_7$.

(8) les céramides naturels ou de synthèse ;
(9) les dihydroxyalkylamines, les amines grasses oxyéthylénées ;
(10) les dérivés du glycérol décrits dans la demande internationale PCT n°91/00889 déposée le 13 Novembre 1991 et répondant à la formule :

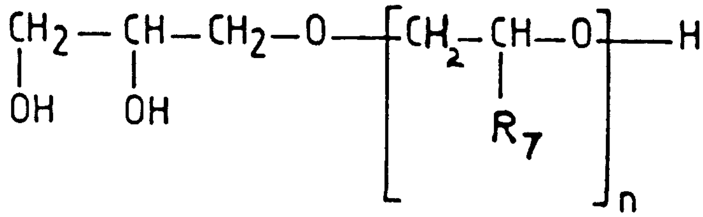
(VI)

formule (VI) dans laquelle $R_7$ représente un radical alkyle linéaire en $C_{14}$ à $C_{18}$ ou un groupement $-CH_2A$ dans lequel A est $-OR_{14}$, $R_{14}$ représentant un radical alkyle linéaire en $C_{10}$-$C_{18}$ et, de préférence, en $C_{16}$, et n représente une valeur statistique moyenne supérieure à 1 et au plus égale à 3 et, en outre, lorsque $R_7$ = $-CH_2A$, n peut également représenter une valeur réelle (non statistique) égale à 2.

B) les lipides amphiphiles ioniques ci-après définis :

(1) les lipides amphiphiles anioniques choisis dans le groupe formé par :

- les phospholipides naturels, notamment la lécithine d'oeuf ou de soja, ou la sphingomyéline, les phospholipides modifiés par voie chimique ou enzymatique, notamment la lécithine hydrogénée, et les phospholipides de synthèse, notamment la dipalmitoylphosphatidylcholine ;
- les composés anioniques, tels que ceux décrits dans le brevet français n° 2 588 256 et représentés par la formule :

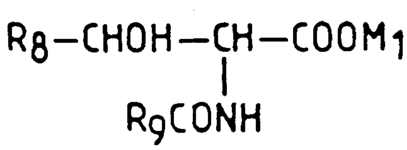
(VII)

formule (VII) dans laquelle :

- . $R_8$ représente un radical alkyle ou alcényle en $C_7$-$C_{21}$
- . $R_9$ représente un radical hydrocarboné, saturé ou insaturé en $C_7$-$C_{31}$, et
- . $M_1$ représente H, Na, K, $NH_4$ ou un ion ammonium substitué dérivé d'une amine,

(2) les composés anioniques choisis dans le groupe formé par :
les esters phosphoriques d'alcools gras, par exemple le dicétylphosphate et le dimyristylphosphate sous forme d'acides ou de sels alcalins ; l'acide heptylnonylbenzène sulfonique ; le sulfate de cholestérol acide et ses

sels alcalins et le phosphate de cholestérol acide et ses sels alcalins ; les lysolécithines ; les alkylsulfates, par exemple le cétyl sulfate de sodium ; les gangliosides ; les lipoaminoacides et/ou lipoamino sels notamment les acylglutamates mono et disodiques ; l'acide phosphatidique et ses sels alcalins ;
(3) les lipides amphiphiles cationiques choisis dans le groupe formé par :

- les composés cationiques dérivés ammonium quaternaire répondant à la formule :

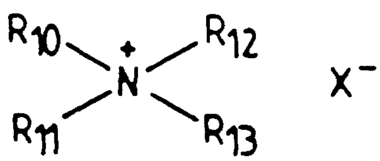 (VIII)

avec $R_{10}$ et $R_{11}$, identiques ou différents, représentant des radicaux alkyle en $C_{12}$ - $C_{20}$ et $R_{12}$ et $R_{13}$, identiques ou différents, des radicaux alkyle en $C_1$ - $C_4$ ;

- les amines à longue chaîne et leurs dérivés ammonium quaternaire, les esters d'aminoalcools à longue chaîne et leurs sels et dérivés ammonium quaternaire ;
- les lipides amphiphiles prépolymérisés obtenus, notamment, à partir des lipides polymérisables donnés page 129 de "Angewandte Chemie" Vol. 27 n° 1, Janvier 1988 ou des produits de réaction d'un lipide anionique et d'un composé cationique polymérisable comme décrit page 137 de la référence ci-dessus.

On peut, de façon connue, ajouter à la phase lipidique vésiculaire différents additifs lipophiles, en particulier des actifs cosmétiques et/ou pharmaceutiques.

De façon connue, également, la phase aqueuse encapsulée des vésicules et/ou la phase aqueuse de dispersion peut contenir des additifs hydrophiles, en particulier des actifs cosmétiques et/ou pharmaceutiques.

La phase aqueuse de dispersion peut, de façon connue, être constituée par de l'eau, ou un mélange d'eau et d'au moins un solvant miscible à l'eau tels que les alcools en $C_1$-$C_7$ et les polyols d'alkyle en $C_1$-$C_5$.

La composition comporte avantageusement une phase grasse associée à la phase aqueuse de dispersion.

Cette phase grasse peut être constituée par au moins une huile sous forme de goutelettes dispersées dans la phase aqueuse de dispersion des vésicules de phase lipidique, la dispersion de gouttelettes étant stabilisée, de façon connue, par les vésicules de phase lipidique ; il est possible également d'utiliser au moins un agent émulsionnant pour stabiliser complémentairement la dispersion de gouttelettes.

Cette phase grasse est, de préférence, sous forme d'émulsion de type eau dans l'huile ou huile dans l'eau, la phase aqueuse de l'émulsion étant au moins partiellement constituée par la phase aqueuse de dispersion des vésicules. La phase grasse peut être toute phase généralement utilisée en cosmétique ou en pharmacie pour la préparation de pommades, crèmes, laits ou lotions utilisables par application topique. Parmi les composés utilisables dans la phase grasse, on peut citer :

- les huiles et corps gras d'origine animale ou végétale formées par des esters d'acide gras et de polyols, en particulier les triglycérides, par exemple les huiles de tournesol, de maïs, de soja, de courge, de pépins de raisin, de jojoba, de sésame, de noisette, les huiles de poisson, le tricaprocaprylate de glycérol, ou les huiles végétales ou animales de formule $R_{14}COOR_{15}$, formule dans laquelle $R_{14}$ représente le reste d'un acide gras supérieur comportant de 7 à 19 atomes de carbone et $R_{15}$ représente une chaîne hydrocarbonée ramifiée contenant de 3 à 20 atomes de carbone, par exemple l'huile de Purcellin ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote ;
- des hydrocarbures, tels que l'hexadécane et l'huile de paraffine ;
- des carbures halogénés, notamment des fluorocarbures tels que des fluoroamines par exemple la perfluorotributylamine, des hydrocarbures fluorés, par exemple le perfluorodécahydronaphtalène, des fluoroesters et des fluoroéthers ;
- des cires ;
- des silicones, par exemple les polysiloxanes, les polydiméthylsiloxanes et les fluorosilicones ;
- des esters d'acide minéral et d'un alcool, et
- des éthers et des polyéthers.

La phase grasse représente généralement de 5 à 50 % en poids par rapport au poids total de la composition.

La composition peut contenir, de façon connue, au moins un actif cosmétique et/ou pharmaceutique, lipophile ou hydrophile. Lorsqu'ils sont lipophiles, les actifs peuvent être introduits dans la phase grasse et/ou, de façon connue, dans la phase lipidique constituant la membrane des vésicules. Lorsque ces actifs sont hydrophiles, ils peuvent être introduits dans la phase aqueuse encapsulée des vésicules et/ou dans la phase aqueuse de dispersion des vésicules. Des actifs amphiphiles peuvent également être utilisés, ces actifs étant susceptibles de se répartir à la fois dans la phase lipidique ou grasse et dans la phase aqueuse.

A titre indicatif, on donne ci-après, dans le tableau I, une liste des actifs utilisables dans les compositions selon l'invention.

TABLEAU I

| FONCTION | ACTIFS UTILISABLES |
|---|---|
| Anti-oxydant ou anti-radicaux libres. | Les extraits des plantes suivantes :<br>- Aubépine.<br>- Ginkgo biloba.<br>- Thé vert.<br>- Vigne.<br>- Romarin<br>Les enzymes<br>- Commercialisées sous la dénomination SB 12 par SEDERMA et constituées par un mélange de lactoferrine et de lactoperoxydase, de glucose oxydase et de thiocyanate de potassium.<br>- Superoxyde dismutase.<br>- Glutathion peroxydase.<br>La superphycodismutase extraite d'algues.<br>Les coenzymes Q, en particulier la coenzyme Q10.<br>Les séquestrants, en particulier des dérivés d'acides polyphosphoniques.<br>Les tanins.<br>Le sélénium et ses dérivés, en particulier la séléniométhionine.<br>Les peptides, par exemple un mélange d'extraits de rate et de thymus,<br>Thiolim et sérum albumine bovine non stabilisée.<br>Les protéines, par exemple l'hémocyanine qui est une protéine cuivrée extraite de l'escargot marin et l'apohémocyanine qui est<br>une protéine analogue sans cuivre.<br>Les flavonoïdes, notamment la catéchine, les proanthocyanidines, les flavanols, les flavones, les isoflavones, les flavanénols,<br>les flavanones, les flavanes et les chalcones.<br>Les caroténoïdes, notamment le β-carotène et le rocou.<br>L'acide sorbohydroxyamique.<br>Les tocophérols, notamment l'alpha-tocophérol et l'acétate d'alpha-tocophérol.<br>Le palmitate d'ascorbyle.<br>Le gallate de propyle.<br>L'acide caféique et ses dérivés.<br>L'acide ascorbique.<br>L'acide homogentisique.<br>L'acide érythorbique.<br>L'acide nordihydroguaïacétique.<br>Le laurylméthionate de lysine.<br>Le butylhydroxyanisole.<br>Le butylhydroxytoluène.<br>Les substances "SOD like". |

TABLEAU I (suite)

| FONCTION | ACTIFS UTILISABLES |
|---|---|
| Hydratant ou humectant | Une reconstitution de sueur ("Normal moisturizing factors"-NMF).<br>Le pyroglutamate de sodium.<br>L'acide hyaluronique.<br>Les dérivés de chitosane (carboxyméthylchitine).<br>Le β-glycérophosphate.<br>Le lactamide.<br>L'acétamide.<br>Le lactate d'éthyle, de sodium et de triéthanolamine.<br>Le pyrrolidone carboxylate de métaux en particulier de Mg, Zn, Fe, Ca ou Na.<br>La thiamorpholinone.<br>L'acide orotique.<br>Les acides carboxyliques alpha-hydroxylés en $C_3$ à $C_{20}$, notamment l'acide alpha-hydroxy propionique.<br>Les polyols, notamment l'inositol, le glycérol, la diglycérine, le sorbitol.<br>Les polyolosides, notamment alginate et guar.<br>Les protéines, notamment le collagène soluble et la gélatine.<br>Les lipoprotides choisis parmi les dérivés mono ou polyacylés d'acides aminés ou de polypeptides dans lesquels le reste acide RCO comporte une chaîne hydrocarbonée en $C_{13}$-$C_{19}$, notamment l'acide palmitoylcaséïnique, l'acide palmitoyl collagénique, le dérivé dipalmitoyl-O-N de l'hydroxyproline, le stéaroyl glutamate de sodium, le stéaroyl tripeptide de collagène, l'oléyltétra et pentapeptide de collagène, le linoléate<br>d'hydroxyproline.<br>L'urée et ses dérivés, notamment la méthylurée.<br>L'extrait de tissu cutané, notamment celui commercialisé sous la<br>dénomination "OSMODYN" par les Laboratoires Serobiologiques de Nancy (LSN) et contenant des peptides, des acides aminés, des saccharides et 17% de mannitol.<br>Plus particulièrement une association de glycérol, d'urée et d'acide<br>palmitoylcaséïnique. |
| Mélanorégulateur :<br>1) accélérateur de bronzage | Les huiles de bergamote et de citrus.<br>L'alpha-MSH et ses homologues synthétiques.<br>La caféine.<br>Les dérivés de tyrosine, notamment le tyrosinate de glucose et la N-malyltyrosine. |

TABLEAU I (suite)

| FONCTION | ACTIFS UTILISABLES |
|---|---|
| 2) Dépigmentant | L'acide ascorbique ou vitamine C et ses dérivés, notamment l'ascorbyle phosphate de Mg.<br>Les hydroxyacides, notamment l'acide glycolique.<br>L'acide kojique.<br>L'arbutine et ses dérivés.<br>L'hémocyanine (protéine cuivrée de l'escargot marin) et l'apohémocyanine (protéine analogue à la précédente sans cuivre).<br>L'hydroquinone et ses dérivés, notamment le monoalkyl éther et le benzyléther. |
| Coloration de la peau (brunissage artificiel) | L'ortho diacétylbenzène.<br>Les indoles.<br>La dihydroxyacétone.<br>L'érythrulose.<br>Le glycéraldéhyde.<br>Les gamma-dialdéhydes, notamment l'aldéhyde tartrique. |
| Liporégulateurs (amincissant et anti-acné, anti-séborrhée) | Les complexes de vitamines et d'oligo-éléments, notamment le complexe vitamine $B_6$/zinc.<br>L'orizanol.<br>L'acide azélaïque.<br>Les xanthines et alkylxanthines, notamment l'extrait de cola, la caféine et la théophylline.<br>L'adénosine monophosphate cyclique et non cyclique.<br>L'adénosine triphosphate.<br>L'extrait de lierre.<br>L'extrait de marron d'Inde.<br>Les extraits d'algues, notamment l'extrait d'algues rouges (fucus serratus) et le cytofiltrat.<br>L'extrait de ginseng.<br>L'extrait de Centella Asiatica (asiaticoside) contenant de la génine et de l'acide asiatique.<br>La thioxolone (HBT).<br>La S-carboxyméthylcystéine.<br>La S-benzylcystéamine. |

TABLEAU I (suite)

| FONCTION | ACTIFS UTILISABLES |
|---|---|
| Antivieillissement et anti-rides | Les insaponifiables par exemple de soja et d'avocats.<br>Les acides gras insaturés, notamment l'acide linoléique et l'acide linolénique.<br>Les hydroxyacides, notamment l'acide glycolique.<br>Les facteurs de croissance.<br>Les complexes oligoéléments - vitamines, notamment $B_6$-Zn.<br>L'acide n-octanoyle-5 salicylique.<br>L'adénosine.<br>Le rétinol et ses dérivés, notamment l'acétate de rétinol et le palmitate de rétinol.<br>Les rétinoïdes, notamment les acides rétinoïques cis ou trans et ceux décrits dans les brevets FR-A-2 570 377 ; EP-A-199636 ; et EP-A-325540 et la demande de brevet européen 90-402072.<br>L'association de rétinoïdes et de xanthines.<br>L'hydroxyproline.<br>Les acides sialiques.<br>L'extrait de rate, de thymus, Thiolim et sérum albumine bovine non stabilisé vendu sous la dénomination commerciale "SILAB" par la Société "SILAB".<br>Un extrait animal placentaire, notamment l'extrait embryonnaire placentaire de bovidés dans l'eau à 5,5 % stabilisé par 0,2 % d'exyl K100a (matrix).<br>Les protéoglycannes, en particulier le protéoglycanne de cartilage de trachée de bovidés à 5 % stabilisé (protéodermin).<br>Le colostrum.<br>Les facteurs d'oxygénation cellulaire, notamment l'octacosamol. |
| Anti-UV | Les filtres UV, notamment le paraméthoxycinnamate d'éthyl-2 hexyle ;<br>la benzophénone,<br>le benzylidène-camphre et leurs dérivés, en particulier, la tétrahydroxy-2,2', 4,4'-benzophénone et l'acide hydroxy-2 méthoxy-4 benzophénone-5 sulfonique ;<br>l'acide paraaminobenzoïque,<br>le salicylate de dipropylèneglycol,<br>l'octyl salicylate,<br>les dérivés de dibenzoylméthane vendus sous les marques EUSOLEX 8020 ou PARSOL 1789 et<br>les produits vendus sous les marques EUSOLEX 232, UNIVUL T 150, UNIVUL N 539, ESCALOL 507. |

TABLEAU I (suite)

| FONCTION | ACTIFS UTILISABLES |
|---|---|
| Kératolytique | L'acide salicylique et ses dérivés, tels que les acides alkylsalicyliques, notamment l'acide n-octanoyl-5 salicylique et le n-dodécanoyl-5, salicylate de N-hexadécyl pyridinium.<br>L'acide rétinoïque.<br>Les enzymes protéolytiques, notamment la trypsine, l'alphachymotrypsine, la papaïne, la bromelaine et la pepsine.<br>Le peroxyde de benzoyle.<br>L'urée.<br>Les alpha-hydroxyacides. |
| Emollient | Esters tels que l'adipate d'isopropyle. |
| Anti-inflammatoire | Les corticoïdes tels que le 17-acétate de β-méthasone, l'indométhacine, le ketoprofène, l'acide flufenamique, l'ibuprofene, le diclofenac, le diflunisal, le fenclofenac, le naproxene, le piroxicam, et le sulindac.<br>Le monostéaryl éther de glycérol (alcool batylique) et le monocétyléther de glycérol (alcool chimylique).<br>L'acide glycyrrhétinique et ses sels, notamment d'ammonium.<br>L'alpha-bisabolol (extrait de camomille).<br>La shikonine.<br>Des extraits de plantes, tels qu'eau de bleuet, arnica, aloès.<br>Des extraits de tissu méristématique, notamment l'extrait de racine de chêne.<br>Le plancton. |
| Rafraichissant | Le menthol.<br>Le lactate de menthyle. |
| Cicatrisant | L'arbre de peau, extrait de mimosa tenui flora.<br>L'extrait de Centella Asiatica.<br>L'acide β-glycyrrhétinique.<br>L'hydroxyproline.<br>L'arginine.<br>Un extrait placentaire.<br>Un extrait de levure.<br>Le fagaramide.<br>La N-acétyl hydroxyproline.<br>L'acide acexamique et ses dérivés. |
| Protecteur vasculaire | Les flavonoïdes, notamment les dérivés de rutine, tels que l'étoxazorutine et le rutine propylsulfonate de sodium.<br>Les extraits végétaux, notamment l'extrait huileux de Ginkgo<br>biloba, l'extrait de marron d'Inde (escine), de lierre (saponines) et de petit houx.<br>Le nicotinate d'alpha-tocophérol. |

TABLEAU I (suite)

| FONCTION | ACTIFS UTILISABLES |
|---|---|
| Anti-bactérien, antifongique | Le bromure de triméthylcétylammonium.<br>L'acide sorbique.<br>Le peroxyde de benzoyle.<br>Le chlorure de cétylpyridinium.<br>Le chlorure de benzalkonium.<br>L'acide parahydroxybenzoïque et ses sels.<br>Le bromo-2 nitro-2 propanediol-1,3.<br>Le trichloro-3,4,4'-carbanilide.<br>Le trichloro-2,4,4'-hydroxy-2 diphényléther.<br>L'acide déhydroacétique.<br>Un extrait de pamplemousse dans la glycérine et le propylène glycol.<br>La chlorhexidine.<br>L'hexetidine.<br>L'hexamidine. |
| Agent insectifuge | Le diméthyltoluamide. |
| Antiperspirant | Le chlorhydrate d'aluminium.<br>Le chlorure d'aluminium.<br>Le complexe lactate de sodium/aluminium chlorhydroxy.<br>Le chlorhydrate de zirconyle. |
| Déodorant | L'oxyde de zinc.<br>Le ricinoléate de zinc.<br>L'éthyl-2 hexanediol-1,3.<br>L'hexachlorophène.<br>Le produit vendu sous la marque "IRGASAN DP 300". |
| Anti-pelliculaire | L'octopyrox.<br>Les omadines.<br>Le goudron de houille.<br>Le 1-hydroxy-4-méthyl-6-(2,4,4-triméthylpentyl)-2-(1H) pyridinone.<br>Le sulfure de sélénium. |
| Anti-chute des cheveux | Les inhibiteurs de glucuronidases.<br>Les mucopolysaccharides.<br>Le nicotinate de méthyle ou d'hexyle.<br>La forskaline.<br>Le minoxidil.<br>Les xanthines.<br>Les rétinoïdes. |
| Colorant capillaire | Les bases et coupleurs d'oxydation.<br>Les colorants directs.<br>Les colorants auto-oxydables. |
| Agent décolorant pour cheveux | Le peroxyde d'hydrogène. |

TABLEAU I (suite)

| FONCTION | ACTIFS UTILISABLES |
|---|---|
| Réducteur pour permanentes | L'acide thioglycolique.<br>La cystéine.<br>La cystéamine.<br>La N-acétyl cystéine.<br>La N-acétyl cystéamine.<br>Le thioglycolate de glycérol. |
| Agent conditionneur pour peau et cheveux | Polymères cationiques, cations. |

En plus des actifs, les compositions peuvent également contenir des adjuvants de formulation, tels que des conservateurs, des pigments, des parfums, des agents de pH ou des agents gélifiants autres que ceux faisant l'objet de l'invention.

Les exemples donnés ci-après, à titre illustratif et permettront de mieux comprendre l'invention.

EXEMPLE 1 : Essais comparatifs

Dans cet essai, on a comparé le pouvoir stabilisant de différents agents stabilisants sur des vésicules de lécithine de soja.

A - Préparation de la dispersion de vésicules stabilisées

On a préparé des vésicules à partir de lécithine de soja vendue sous la dénomination commerciale "NATIPIDE II" par la société "NATTERMANN" par dispersion de 25 % en poids de "NATIPIDE II" dans de l'eau distillée à la température de 25° C. On a obtenu après 30 mn d'agitation modérée au barreau magnétique une dispersion dans une phase aqueuse de vésicules dont la taille moyenne, mesurée à l'aide d'un granulomètre laser commercialisé sous la dénomination "AMTECH BI 90", est de 200 nm avec une polydispersité de 0,1. Indépendamment, on a préparé une solution à 1 % en poids d'agent stabilisant dans de l'eau distillée. On a mélangé la dispersion de vésicules et la solution d'agent stabilisant de façon à avoir une composition contenant (en poids) 5 % de lécithine de soja et 0,1 % d'agent stabilisant. Le mélange est effectué en 1 heure sous agitation modérée à l'aide d'un barreau magnétique.

B - Test

On ajoute ensuite un agent tensioactif à une température supérieure à la température de fusion du tensioactif, de façon à avoir dans le mélange une concentration finale de 1 % en poids de tensioactif. On agite ensuite vigoureusement le mélange pendant 30 secondes à l'aide d'un homogénéiseur de type "VORTEX". Après retour à la température ambiante, chaque mélange est soumis pendant 3 minutes à l'action des ultrasons générés par la microsonde d'un appareil commercialisé sous la dénomination "BRANSON" de type B 30 avec le réglage suivant :

- cycle de travail : 50 %
- puissance : position 6.

La température du mélange est maintenue proche de la température ambiante pendant l'action des ultrasons à l'aide d'un bain-marie d'eau glacée.

On mesure ensuite la turbidité de chaque mélange, après dilution au 1/150, à la longueur d'onde de 400 nm à l'aide d'un spectrophotomètre UV visible. Sous l'action des tensioactifs, la turbidité baisse.

Les mesures ont été effectuées avec les tensioactifs commercialisés sous les dénominations suivantes:

- "BRIJ 72", alcool portant 2 unités oxyde d'éthylène commercialisé par la société "ICI" ;
- "TWEEN 60", polysorbate commercialisé par la société "ICI" ;
- "TWEEN 20", polysorbate commercialisé par la société "ICI" ;
- "LSS", lauryl sulfate de sodium commercialisé par la société "RHONE-POULENC" ;
- "PF 68", copolymère polyoxyéthylène/polyoxypropylène commercialisé par la société "ICI" ;

- "MYRJ 52", stéarate de polyoxyéthylène commercialisé par la société "ICI" ;
- "ARLAMOL E", alcool polypropyléné commercialisé par la société "ICI" ;
- "TEGIN 90", stéarate de glycérol commercialisé par la société "GOLDSCHMIDT".

Pour chaque tensioactif, les essais ont été effectués sans addition d'agent stabilisant (vésicules non stabilisées) et avec les agents stabilisants suivants :

1) Composés ne faisant pas partie de l'invention :

- Chitosane,
- ADN de haut poids moléculaire commercialisé par "JAVENECH",
- Alginate de sodium,
- Gomme arabique,
  composé commercialisé sous la dénomination commerciale "ETICANE 3" (mélange d'atélocollagène et de glycoaminoglycanne) par la société "BIOETICA",

2) Composés faisant partie de l'invention :

- Alginates de propylèneglycol estérifiés à 80-85 % commercialisés par la société "KELCO" sous les dénominations "KELCOLOID O" et "MANUCOLESTER E/PL",
- Gommes de gélane commercialisées sous les dénominations "KELCOGEL" et "KELCOGEL PC" par la société "KELCO", et
- Gomme de wélane commercialisée sous la référence "K1A96" par la société "KELCO".

Le tableau II ci-après donne d'une part, la baisse de turbidité en % calculée par rapport à un témoin ne contenant ni tensioactif, ni stabilisant et, d'autre part, l'indice de protection à 80 % déterminé à partir des données de baisse de turbidité. L'indice de protection à 80 % correspond au rapport (nombre d'agents tensioactifs testés dans lesquels la baisse de turbidité a été inférieure à 20 % par rapport à celle du témoin/nombre total d'agents tensioactifs testés). La baisse de turbidité donnée dans le tableau II est la moyenne de trois essais.

## TABLEAU II

| TENSIO-ACTIF | VESICULES NON STABILISEES | CHITOSANE | ADN | ALGINATE Na+ | GOMME ARABIQUE | ETICANE 3 | KELCOLOID 0 | KELCOGEL | GOMME de WELANE | MANUCOL-ESTER E/PL | KELCOGEL PC |
|---|---|---|---|---|---|---|---|---|---|---|---|
| BRIJ 72 | -40% ±1 | D(1) | -31% ±3 | -10% ±0 | -11% ±1 | -17% ±2 | +8% ±3 | 0% ±1 | +7% ±1 | +5% ±1 | +5% ±3 |
| TWEEN 60 | -56% ±2 | D | -34% ±3 | -33% ±2 | -31% ±3 | -29% ±2 | +4% ±5 | 0% ±1 | -2% ±3 | 0% ±1 | +5% ±1 |
| TWEEN 20 | -69% ±2 | D | -60 ±10 | -57% ±3 | -54% ±1 | -54% ±3 | 0% ±7 | 0% ±8 | -10% ±3 | -14% ±2 | -5% ±4 |
| LSS | -68% ±3 | D | -64% ±2 | -59% ±6 | -50% ±1 | -58% ±6 | -6% ±8 | -7% ±8 | -8% ±2 | -13% ±2 | -5% ±4 |
| PF 68 | -38% ±1 | D | -39% ±8 | -30% ±9 | -16% ±1 | -30% ±1 | 0% ±1 | 0% ±1 | +3% ±1 | 0% ±2 | +1% ±1 |
| MYRJ 52 | -48% ±2 | D | -48% ±3 | -44% ±5 | -41% ±5 | -47% ±1 | -5% ±2 | -7% ±3 | +4% ±2 | -3% ±3 | +1% ±1 |
| ARLAMOL E | -31% ±3 | D | -11% ±3 | -15% ±1 | -13% ±2 | -4% ±4 | +2% ±2 | +3% ±2 | - | - | - |
| STEARATE de GLYCEROL | -19% ±1 | D | -18% ±1 | -6% ±3 | -6% ±6 | -11% ±2 | -2% ±2 | -2% ±3 | - | - | - |
| INDICE de PROTECTION à 80% | | 0/8 | 0/8 | 1/8 | 0/8 | 1/8 | 8/8 | 8/8 | 6/6 | 6/6 | 6/6 |

(1) : D déstabilisation des vésicules ; floculation en amas irréversibles ; cristaux et grossissement.

EXEMPLE 2 :

Dans cet exemple, les mêmes essais que dans l'exemple 1 ont été effectués avec des doses variables d'agents stabilisants selon l'invention. Les résultats sont rassemblés dans le tableau III ci-après.

Ces essais montrent qu'avec une concentration en agent stabilisant selon l'invention de 0,10 %, c'est-à-dire une proportion pondérale (agent stabilisant/phase lipidique) de 2 %, on obtient d'excellents résultats de stabilisation et qu'avec des concentrations de 0,05 et 0,01 %, c'est-à-dire avec une proportion pondérale de 1 et 0,2 % par rapport à la phase lipidique vésiculaire, on obtient encore une protection sensible.

## TABLEAU III

| TENSIOACTIF | KELCOLOID 0 | | | KELCOGEL | | | VESICULES NON STABILISEES |
|---|---|---|---|---|---|---|---|
| | PROPORTION PONDERALE PAR RAPPORT AU POIDS TOTAL DE LA COMPOSITION | | | | | | |
| 1 % | 0,10% | 0,05% | 0,01% | 0,10% | 0,05% | 0,01% | 0% |
| BRIJ 72 | +8%±3 | +1%±1 | 0% | 0%±1 | 0% | 0% | -39%±2 |
| TWEEN 60 | +4%±5 | -9%±1 | -13%±2 | 0%±1 | -8%±1 | -15%±2 | -51%±1 |
| TWEEN 20 | 0%±7 | -26%±2 | -35%±2 | 0%±8 | -25%±1 | -42%±2 | -68%±1 |
| LSS | -6%±8 | -17%±1 | -32%±2 | -7%±8 | -12%±3 | -33%±2 | -66%±3 |
| PF 68 | 0%±1 | +1%±1 | -6%±1 | 0%±1 | 0%±1 | -5%±4 | -36%±1 |
| MYRJ 52 | -5%±2 | -5%±4 | -14%±1 | -7%±3 | -5%±2 | -22%±1 | -52%±1 |

EXEMPLE 3

Dans cet exemple, le même essai que celui réalisé dans l'exemple 1 a été effectué avec des vésicules de lipide non-ionique.

A - Préparation de la dispersion de vésicules stabilisées

On a préparé des vésicules à partir :

- d'un lipide amphiphile non-ionique de formule (A) ci-après :

$$R-CO\{O-C_2H_3-O\{CH_2-O-CH_2-CHOH-CH_2OH\}\}H \qquad (A)$$

dans laquelle :

- R représente un reste isostéaryle ($C_{18}$ ramifié), et
- $-O-C_2H_3-O-$ représente les structures suivantes prises en mélange ou séparément :

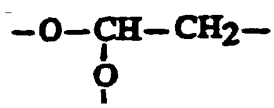 et 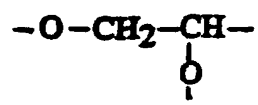

- et du sel monosodique de l'acide L-N-stéaroylglutamique, commercialisé par la société "AJINOMOTO" sous la dénomination commerciale "ACYLGLUTAMATE HS 11", dans un rapport pondéral 95/5.

Ces deux composés lipidiques sont mélangés par fusion, puis hydratés à chaud (40°C) par de l'eau distillée. Le mélange lipidique représente 5 % en poids par rapport au poids total de la dispersion. La température est ramenée à 25° C. On a obtenu après 10 minutes d'agitation, à l'aide d'un homogénéisateur de type commercialisé sous la dénomination VIRTIS 60", dont la vitesse est de 40 000 t/mn, une dispersion de vésicules dans une phase aqueuse. La taille des vésicules obtenues est comprise entre 100 et 150 nm.

Indépendamment, on a préparé une solution à 1 % en poids d'agent stabilisant dans de l'eau distillée. On a mélangé la dispersion de vésicules et la solution d'agent stabilisant de façon à avoir une composition contenant 5 % en poids du mélange lipidique décrit ci-dessus et 0,1 % en poids d'agent stabilisant. Le mélange est effectué en 1 heure sous agitation moyenne à l'aide d'un barreau aimanté.

B - Test

On procède ensuite comme indiqué à l'exemple 1.
Les résultats figurent dans le tableau IV ci-après :

TABLEAU IV

| Tensioactifs | Vésicules non stabilisées | KELCOLOID O | KELCOGEL PC |
|---|---|---|---|
| TWEEN 60 | - 11 % | - 5 % ± 1 | + 3 % ± 3 |
| TWEEN 20 | - 21 % | - 3 % ± 1 | + 2 % ± 2 |
| LSS | - 38 % | - 7 % ± 1 | - 7 % ± 2 |
| PF 68 | - 31 % | 0 % ± 1 | 0 % ± 1 |
| MYRJ 52 | - 35 % | - 5 % ± 2 | - 4 % ± 1 |
| Protection à 80% | | 6/6 | 6/6 |

EXEMPLE 4 : Emulsion de type huile dans eau (H/E)

Dans un bêcher de 200 ml, on dissout :

- 5 g d'alcool cétylique,
- 3 g d'un mélange de mono et distéarate de glycérol commercialisé sous la dénomination commerciale "GELEOL COPEAUX",
- 3 g de stéarate de polyoxyéthylène commercialisé sous la dénomination commerciale "MYRJ 53" par la société "ICI"

dans 24 g d'huile de vaseline chauffée à la température de 65°C.
Après avoir ramené la température à 50°C, on ajoute, sous l'agitation donnée par un homogénéiseur commercialisé sous la dénomination "MORITZ", une phase aqueuse à la même température constituée d'une solution de 0,3 g du mélange de conservateur commercialisé sous la dénomination commerciale "ELASTAB 4112" par la société "LSN" dans 54,7 g d'eau. L'homogénéisation est maintenue pendant le refroidissement du produit à la température ambiante. On ajoute alors une suspension de vésicules constituées de :

- 2,5 g de lécithine de soja commercialisée sous la dénomination commerciale "NATIPIDE II" par la société "NATTERMAN",
- 7,49 g d'eau,
- 0,01 g d'alginate de propylène glycol commercialisé par la société "KELCO" sous la dénomination commerciale "KELCOLOID O".

On obtient ainsi 100 g d'une crème épaisse destinée au soin des peaux sèches. On a constaté, par microscopie optique, la présence de vésicules après 3 mois de stockage.

EXEMPLE 5 : Emulsion de type eau dans l'huile (E/H)

Dans un bêcher en verre de 200 ml, on mélange à la température de 85°C à l'aide d'un homogéiseur de type "MORITZ" :

- 5,7 g d'un mélange de lanolate de magnésium et d'huile de vaseline (50/50) commercialisé sous la dénomination commerciale "MEXANYL 60" par la société "CHIMEX",
- 6,65 g de lanoline hydrogénée commercialisée sous la dénomination commerciale "SUPERSAT" par la société "RITA", et
- 2,0 g de 2-éthylhexyl éther de l'ester de glycérine et d'acide palmitique (octoxyglycéryl palmitate) commercialisé sous la dénomination commerciale "MEXANYL GP" par la société "CHIMEX".

Après homogénéisation, ce premier mélange est dissout dans un second mélange constitué de :

- 3 g d'huile de Purcellin liquide,
- 3 g d'un mélange d'alcool de lanoline et d'huile de vaseline (15/85) commercialisé sous la dénomination commerciale "LIQUIDE BASE CB 1145" par la société "CRODA",
- 4,75 g de palmitate d'isopropyle,
- 7,9 g d'huile de vaseline,
- 15 g de vaseline blanche, et
- 0,4 g de perhydrosqualène.

Ces deux mélanges sont homogénéisés à la température de 80°C, puis refroidis à 40°C.
Sous agitation, et à la température de 40°C, on introduit ensuite la phase aqueuse constituée par le mélange d'une solution de :

- 0,25 g du mélange d'agents conservateurs commercialisé sous la dénomination commerciale d'"ELASTAB 4110" par la société "LSN" dans 39,9 g d'eau, et
- 10 g d'une dispersion vésiculaire constituée de :
  - 2,5 g de lécithine de soja commercialisée sous la dénomination commerciale "NATIPIDE II" par la société "NATTERMAN",
  - 0,02 g de gomme de gélane commercialisée sous la dénomination commerciale "KELCOGEL PC" par la société "KELCO" et de
  - 7,8 g d'eau.

On maintient sous agitation pendant 20 minutes et on ajoute alors le mélange formé par :

- 0,3 g d'un conservateur commercialisé sous la dénomination commerciale "GERMAL 115", et
- 0,35 g d'eau.

Après retour à la température ambiante, on passe le produit obtenu sur une broyeuse à trois cylindres. On obtient ainsi une crème blanche, lisse, épaisse, destinée au soin des peaux très sèches. On a constaté, par microscopie optique, la présence de vésicules après 3 mois de stockage.

EXEMPLE 6 :Lait de toilette très doux

Dans un bêcher de 200 ml, on dissout :

- 2,8 g de glycéryl stéarate commercialisé sous la dénomination commerciale "SIMULSOL 165" par la société "SEPPIC",
- 0,75 g d'alcool stéarylique ;
  dans un mélange d'huiles porté à 65°C et composé de :
  - 12 g d'huile de vaseline,
  - 0,5 g d'huile de lanoline commercialisée sous la dénomination commerciale "ARGONOL 60" par la société "WESTBROOCK",
  - 2 g d'huile d'amande douce commercialisée par la société "SICTIA",
  - 0,05 g de parahydroxybenzoate de propyle.

On ajoute, sous l'agitation donnée par un homogénéiseur "MORITZ", une phase aqueuse à la même température constituée d'une solution de :

- 0,2 g de parahydroxybenzoate de méthyle,
- 5 g de glycérine,
- 0,05 g de conservateur commercialisé sous la dénomination commerciale "GERMAL 115",
- 0,1 g d'homopolymère d'acide acrylique réticulé avec un allyl éther de pentaérythritol ou un allyl éther de sucrose, commercialisé sous la dénomination commerciale "CARBOPOL 941" par la société "GOODRICH",
- 0,13 g de triéthanolamine, et
- 66,42 g d'eau.

L'homogénéisation est maintenue pendant 20 minutes, puis on agite, avec une vitesse lente des pales, à l'aide d'un agitateur de type "RAYNERI" jusqu'au refroidissement complet de la formule. On ajoute alors, sous agitation réduite, une dispersion vésiculaire constituée de :

- 2,5 g de lécitine de soja commercialisée sous la dénomination commerciale "NATIPIDE II" par la société "NATTERMAN",
- 7,49 g d'eau,
- 0,01 g d'alginate de propylène glycol commercialisé sous la dénomination commerciale "MANUCOL ESTER E/PL" par la société "KELCO".

On obtient alors un lait très doux, blanc. On a constaté, par microscopie optique, la présence de vésicules après 3 mois de stockage.

EXEMPLE 7 : Crème de nuit (E/H)

Dans un bêcher de 200 ml, on dissout dans 20 g d'huile de vaseline à 65°C :

- 5 g d'isostéarate de sorbitan commercialisé sous la dénomination commerciale "ARLACEL 987" par la société "ICI",
- 1,3 g de cire commercialisée sous la dénomination commerciale "DEA WAX 74181" par la société "CONDEA",
- 1,5 g de paraffine commercialisée sous la dénomination commerciale "CERAFINE 56/58 par la société "CERESINE",
- 5 g d'un mélange de triglycérides d'acides caprique et caprylique, d'hectorite de stéaralkonium et de carbonate de propylène commercialisé sous la dénomination commerciale "MIGLYOL GEL B" par la société "HULS",

- 0,2 g de parahydroxybenzoate de propyle.

Sous agitation d'un homogénéiseur de type "MORITZ" et à 40°C, on introduit ensuite une phase aqueuse constituée par le mélange de :

- 3 g de glycérine,
- 0,5 g de sulfate de magnésium ($7H_2O$),
- 0,25 g d'un conservateur commercialisé sous la dénomination commerciale "GERMAL 115",
- 0,2 g de parahydroxybenzoate de méthyle,

dans 54,55 g d'eau, ainsi qu'une dispersion vésiculaire constituée de :

- 2,5 g de lécithine de soja commercialisée sous la dénomination commerciale "NATIPIDE II" par la société "NATTERMAN",
- 7,49 g d'eau,
- 0,01 g de gomme de wélane commercialisée sous la dénomination commerciale "$K1_A96$" par la société "KELCO".

On obtient alors une crème blanche, lisse, épaisse, destinée aux peaux très sèches. On a constaté, par microscopie optique, la présence de vésicules après 3 mois de stockage.

<u>EXEMPLE 8</u> : Crème de jour hydratante (émulsion H/E)

Dans un bêcher de 200 ml, on dissout dans 18 g d'huile de vaseline à 80°C :

- 7 g de cire d'abeille,
- 2 g de glycéryl stéarate commercialisé sous la dénomination commerciale "TEGIN" par la société "GOLDSCHMIDT",
- 4 g d'huile de lanoline commercialisée sous la dénomination commerciale "AMERCHOL L101" par la société "AMERCHOL",
- 5 g de myristate d'isopropyle,
- 0,5 g de cétylphosphate de potassium commercialisé sous la dénomination commerciale "AMPHISOL K" par la société "GIVAUDAN-ROURE",

On ajoute, sous l'agitation donnée par un homogénéiseur commercialisé sous la dénomination commerciale "MORITZ", une phase aqueuse à la même température constituée de :

- 0,2 g de parahydroxybenzoate de méthyl,
- 0,3 g de polymère acrylique réticulé commercialisé sous la dénomination commerciale "CARBOMER 940" par la société "GOODRICH",
- 5 g de glycérine,
- 0,3 g de conservateur commercialisé sous la dénomination commerciale "GERMAL 115",
- 0,3 g de triéthanolamine,
- 47,4 g d'eau.

On maintient l'agitation jusqu'à 25°C puis on y ajoute, sous l'agitation correspondant à une vitesse lente des pales d'un agitateur de type commercialisé sous la dénomination commerciale "RAYNERI", une dispersion vésiculaire composée de :

- 0,9 g du lipide amphiphile non-ionique de formule (A) de l'exemple 3,
- 0,1 g du composé commercialisé sous la dénomination commerciale d' "ACYL GLUTAMATE HS 11" par la société "AJINOMOTO",
- 0,02 g de gomme de gélane commercialisée sous la dénomination "KELCOGEL" par la société "KELCO",
- 8,98 g d'eau.

Après complète dispersion, on obtient une crème blanche pour le soin quotidien des peaux sèches. On a constaté, par microscopie optique, la présence de vésicules après 3 mois de stockage.

EXEMPLE 9 : Crème pour peaux sèches (émulsion H/E)

Dans un bêcher de 200 ml, on dissout :

- 5 g d'alcool cétylique,
- 3 g d'un mélange de mono et distéarate de glycérol, commercialisé sous la dénomination commerciale "GELEOL COPEAUX",
- 3 g de stéarate de polyoxyéthylène, commercialisé sous la dénomination commerciale "MYRJ 53" par la société "ICI"

dans 24 g d'huile de vaseline chauffée à la température de 65°C.

Après avoir ramené la température à 50°C, on ajoute, sous l'agitation donnée par un homogénéiseur commericalisé sous la dénomination commerciale "MORITZ", une phase aqueuse à la même température constituée d'une solution de 0,3 g du mélange de conservateur commercialisé sous la dénomination commerciale "ELASTAB 4112" par la société "LSN" dans 54,7 g d'eau. L'homogénéisation est maintenue pendant le refroidissement du produit à la température ambiante. On ajoute alors une suspension de vésicules constituées de :

- 2,5 g de lécithine de soja commercialisée sous la dénomination commerciale "NATIPIDE II" par la société "NATTERMAN",
- 7,49 g d'eau,
- 0,01 g d'alginate de glycérol commercialisé par la société "KELCO".

On obtient ainsi 100 g d'une crème épaisse destinée au soin des peaux sèches. On a constaté, par microscopie optique, la présence de vésicules après 3 mois de stockage.

## Revendications

1. Procédé de stabilisation de vésicules formées d'une membrane de phase lipidique contenant au moins un lipide amphiphile ionique et/ou non-ionique encapsulant une phase aqueuse, sous forme de dispersion dans une phase aqueuse, par addition dans la phase aqueuse de dispersion d'au moins un agent stabilisant, caractérisé par le fait que l'(les) agent(s) stabilisant(s) est(sont) choisi(s) dans le groupe formé par les alginates de glycérol, les alginates de propylèneglycol, la gomme de gélane et la gomme de wélane.

2. Procédé selon la revendication 1, caractérisé par le fait que l'agent stabilisant est un alginate estérifié à plus de 60 % par du propylèneglycol ou du glycérol.

3. Procédé selon l'une des revendications 1 ou 2, caractérisé par le fait que l'(les) agent(s) stabilisant(s) est (sont) utilisé(s) à raison de 0,1 % à 20 % en poids par rapport au poids de la phase lipidique vésiculaire.

4. Procédé selon la revendication 3, caractérisé par le fait que l'(les) agent(s) stabilisant(s) est (sont) utilisé(s) à raison de 0,2 % à 10 % en poids par rapport au poids de la phase lipidique vésiculaire.

5. Procédé selon l'une des revendications 1 à 4, caractérisé par le fait que la composition contient jusqu'à 20 % en poids d'agent(s) tensioactif(s) par rapport au poids total de la composition.

6. Procédé selon l'une des revendications 1 à 5, caractérisé par le fait que la phase aqueuse de dispersion est associée à une phase grasse, la phase grasse représentant jusqu'à 50 % en poids de la phase aqueuse de dispersion.

7. Composition pour application topique contenant, en premier lieu, une dispersion dans une phase aqueuse de vésicules formées d'une membrane de phase lipidique contenant au moins un lipide amphiphile ionique et/ou non-ionique encapsulant une phase aqueuse et, en second lieu, au moins un agent stabilisant desdites vésicules, caractérisée par le fait que l'(les) agent(s) stabilisant(s) est(sont) choisi(s) dans le groupe formé par les alginates de glycérol, les alginates de propylèneglycol, la gomme de gélane et la gomme de wélane.

8. Composition selon la revendication 7, caractérisée par le fait que l'(les) agent(s) stabilisant(s) représente(nt) de 0,1 à 20% en poids par rapport au poids de la phase lipidique vésiculaire.

**9.** Composition selon la revendication 8, caractérisée par le fait que l'(les) agent(s) stabilisant(s) représente(nt) de 0,2% à 10 % en poids par rapport au poids de la phase lipidique vésiculaire.

**10.** Composition selon l'une des revendications 7 à 9, caractérisée par le fait que la composition comprend de 1 à 20 % en poids d'agent(s) tensioactif(s) par rapport au poids total de la composition.

**11.** Composition selon la revendication 10, caractérisée par le fait que l'agent tensioactif est choisi dans le groupe formé par les alkylsulfates de sodium, les esters de polyol et les esters de polyol oxyéthylénés, les alcools polyoxyéthylénés, les alcools polypropylénés et les copolymères polyoxyéthylène/polyoxypropylène.

**12.** Composition selon l'une des revendications 7 à 11, caractérisée par le fait que la phase lipidique vésiculaire comprend au moins un lipide choisi dans le groupe formé par :

A) les lipides non-ioniques ci-après définis :

(1) les dérivés du glycérol, linéaires ou ramifiés, de formule

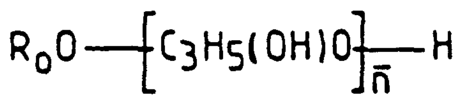 (IV)

formule (IV) dans laquelle :

. $-C_3H_5(OH)O-$ est représenté par les structures suivantes prises en mélange ou séparément :

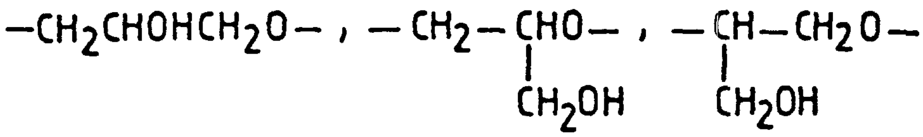

. n est une valeur statistique moyenne comprise entre 1 et 6 ou bien n = 1 ou 2 et alors $-C_3H_5(OH)O-$ est représenté par la structure $-CH_2CHOH-CH_2O-$ ;
. $R_o$ représente :

(a) une chaîne aliphatique, linéaire ou ramifiée saturée ou insaturée, contenant de 12 à 30 atomes de carbone ; ou des radicaux hydrocarbonés des alcools de lanoline ; ou les restes d'alpha-diols à longue chaîne ;
(b) un reste $R_1CO$, où $R_1$ est un radical aliphatique, linéaire ou ramifié, en $C_{11}$-$C_{29}$ ;
(c) un reste

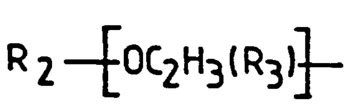

où :

. $R_2$ peut prendre la signification (a) ou (b) donnée pour $R_o$ ;
. $-OC_2H_3(R_3)-$

est représenté par les structures suivantes, prises en mélange ou séparément :

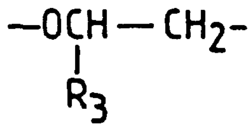 et 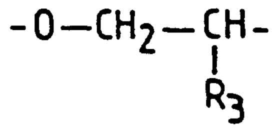

où $R_3$ prend la signification (a) donnée pour $R_o$ ;
(2) les éthers de polyglycérol, linéaires ou ramifiés, comportant deux chaînes grasses ;
(3) les diols à chaîne grasse ;
(4) les alcools gras oxyéthylénés ou non, les stérols et les phytostérols, oxyéthylénés ou non ;
(5) les éthers et esters de polyols, oxyéthylénés ou non, l'enchaînement des oxydes d'éthylène pouvant être linéaire ou cyclique ;
(6) les glycolipides d'origine naturelle ou synthétique, les éthers et esters de mono ou polysaccharides et notamment les éthers et les esters de glucose ;
(7) les hydroxyamides représentés par la formule :

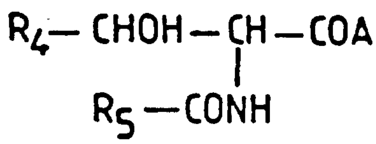 (V)

formule (V) dans laquelle :

- $R_4$ désigne un radical alkyle ou alcényle en $C_7$-$C_{21}$ ;
- $R_5$ désigne un radical hydrocarboné, saturé ou insaturé, en $C_7$-$C_{31}$;
- COA désigne un groupement choisi parmi les deux groupements suivants :

  . un reste

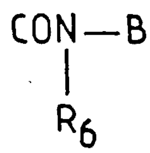

  où :

  . B est un radical alcoyle dérivé d'amines primaires ou secondaires, mono- ou polyhydroxylées ; et
  . $R_6$ désigne un atome d'hydrogène ou un radical méthyle, éthyle ou hydroxyéthyle ; et
  . un reste -COOZ, où Z représente le reste d'un polyol en $C_3$-$C_7$.

(8) les céramides naturels ou de synthèse ;
(9) les dihydroxyalkylamines, les amines grasses oxyéthylénées ;
(10) les dérivés du glycérol répondant à la formule :

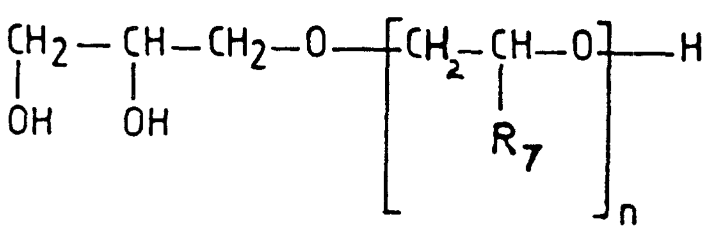 (VI)

formule (VI) dans laquelle $R_7$ représente un radical alkyle linéaire en $C_{14}$ à $C_{18}$ ou un groupement $-CH_2A$

dans lequel A est $-OR_{14}$, $R_{14}$ représentant un radical alkyle linéaire en $C_{10}$-$C_{18}$ et, de préférence, en $C_{16}$, et n représente une valeur statistique moyenne supérieure à 1 et au plus égale à 3 et, en outre, lorsque $R_7 = -CH_2A$, n peut également représenter une valeur réelle (non statistique) égale à 2.

B) les lipides amphiphiles ioniques ci-après définis :

(1) les lipides amphiphiles anioniques choisis dans le groupe formé par :

- les phospholipides naturels, les phospholipides modifiés par voie chimique ou enzymatique et les phospholipides de synthèse ;
- les composés anioniques représentés par la formule

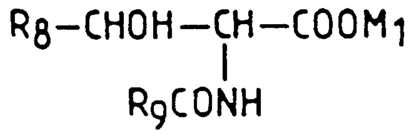

(VII)

formule (VII) dans laquelle :

. $R_8$ représente un radical alkyle ou alcényle en $C_7$-$C_{21}$
. $R_9$ représente un radical hydrocarboné, saturé ou insaturé en $C_7$-$C_{31}$, et
. $M_1$ représente H, Na, K, $NH_4$ ou un ion ammonium substitué dérivé d'une amine,

(2) les composés anioniques choisis dans le groupe formé par : les esters phosphoriques d'alcools gras ; l'acide heptylnonylbenzène sulfonique ; le sulfate de cholestérol acide et ses sels alcalins et le phosphate de cholestérol acide et ses sels alcalins ; ; les lysolécithines ; les alkylsulfates ; les gangliosides ;
(3) les lipides amphiphiles cationiques choisis dans le groupe formé par :

- les composés cationiques dérivés ammonium quaternaire répondant à la formule :

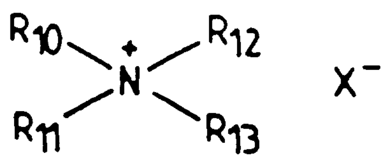

(VIII)

avec $R_{10}$ et $R_{11}$, identiques ou différents, représentant des radicaux alkyle en $C_{12}$ - $C_{20}$ et $R_{12}$ et $R_{13}$, identiques ou différents, des radicaux alkyle en $C_1$ - $C_4$ ;

- les amines à longue chaîne et leurs dérivés ammonium quaternaire, les esters d'aminoalcools à longue chaîne et leurs sels et dérivés ammonium quaternaire ;

- les lipides amphiphiles prépolymérisés obtenus à partir des lipides polymérisables ou par réaction d'un lipide anionique et d'un composé cationique polymérisable.

**13.** Composition selon l'une des revendications 7 à 12, caractérisée par le fait que la phase aqueuse de dispersion est constituée par de l'eau ou un mélange d'eau et d'au moins un alcool en $C_1$-$C_7$ et/ou un polyol d'alkyle en $C_1$-$C_5$.

**14.** Composition selon l'une des revendications 7 à 13, caractérisée par le fait qu'une phase grasse est associée à la phase aqueuse de dispersion.

**15.** Composition selon la revendication 14, caractérisée par le fait que la phase grasse est sous forme d'émulsion de type eau dans l'huile ou huile dans l'eau, la phase aqueuse de l'émulsion étant constituée par la phase aqueuse de dispersion des vésicules.

**16.** Composition selon l'une des revendications 14 ou 15, caractérisée par le fait que la phase grasse est constituée

par au moins un composé choisi dans le groupe constitué par les huiles et corps gras d'origine animale ou végétale, les huiles essentielles naturelles ou synthétiques, les cires, les hydrocarbures, les carbures halogénés, les silicones, les esters d'acide minéral et d'un alcool, et les éthers et polyéthers.

**17.** Composition selon l'une des revendications 14 à 16, caractérisée par le fait que la phase grasse représente de 5 à 50 % en poids par rapport au poids total de la composition.

**18.** Composition selon l'une des revendications 7 à 17, caractérisée par le fait qu'elle contient au moins un actif cosmétique et/ou pharmaceutique.

**19.** Composition selon l'une des revendications 7 à 18, caractérisée par le fait qu'elle contient au moins un adjuvant de formulation.

**Claims**

**1.** Process for stabilizing vesicles formed of a membrane having a lipid phase containing at least one ionic and/or nonionic amphiphilic lipid encapsulating an aqueous phase, in the form of a dispersion in an aqueous phase, by adding to the aqueous dispersing phase at least one stabilizing agent, characterized in that the stabilizing agent (s) is(are) chosen from the group consisting of glyceryl alginates, propylene glycol alginates, gelan gum and welan gum.

**2.** Process according to Claim 1, characterized in that the stabilizing agent is an alginate which is more than 60% esterified with propylene glycol or glycerol.

**3.** Process according to either of Claims 1 and 2, characterized in that the stabilizing agent(s) is(are) used in an amount of 0.1% to 20% by weight relative to the weight of the vesicular lipid phase.

**4.** Process according to Claim 3, characterized in that the stabilizing agent(s) is(are) used in an amount of 0.2% to 10% by weight relative to the weight of the vesicular lipid phase.

**5.** Process according to one of Claims 1 to 4, characterized in that the composition contains up to 20% by weight of surfactant(s) relative to the total weight of the composition.

**6.** Process according to one of Claims 1 to 5, characterized in that the aqueous dispersing phase is combined with a fatty phase, the fatty phase representing up to 50% by weight of the aqueous dispersing phase.

**7.** Composition for topical application containing, firstly, a dispersion of vesicles formed of a membrane having a lipid phase containing at least one ionic and/or nonionic amphiphilic lipid encapsulating an aqueous phase, which are dispersed in an aqueous phase, and secondly, at least one agent stabilizing the said vesicles, characterized in that the stabilizing agent(s) is (are) chosen from the group formed by glyceryl alginates, propylene glycol alginates, gelan gum and welan gum.

**8.** Composition according to Claim 7, characterized in that the stabilizing agent(s) represent(s) from 0.1 to 20% by weight relative to the weight of the vesicular lipid phase.

**9.** Composition according to Claim 8, characterized in that the stabilizing agent(s) represent(s) from 0.2 to 10% by weight relative to the weight of the vesicular lipid phase.

**10.** Composition according to one of Claims 7 to 9, characterized in that the composition comprises from 1 to 20% by weight of surfactant(s) relative to the total weight of the composition.

**11.** Composition according to Claim 10, characterized in that the surfactant is chosen from the group consisting of sodium alkyl sulphates, polyol esters and oxyethylenated polyol esters, polyoxyethylenated alcohols, polypropylenated alcohols and polyoxyethylene/polyoxypropylene copolymers.

**12.** Composition according to one of Claims 7 to 11, characterized in that the vesicular lipid phase comprises at least one lipid chosen from the group formed by:

A) the nonionic lipids defined below:

(1) the linear or branched derivatives of glycerol of formula

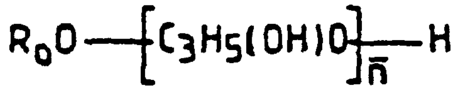 (IV)

in which formula (IV):

- $-C_3H_5(OH)O-$ is represented by the following structures taken as a mixture or separately:

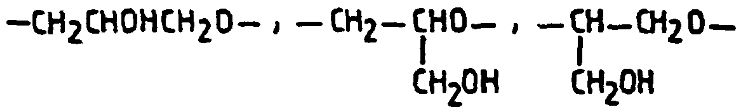

- n is a mean statistical value between 1 and 6 or n = 1 or 2 and then $-C_3H_5(OH)O-$ is represented by the structure $-CH_2CHOH-CH_2O-$;
- $R_0$ represents:

(a) a saturated or unsaturated, linear or branched aliphatic chain containing from 12 to 30 carbon atoms; or hydrocarbon radicals of lanolin alcohols; or long-chain alpha-diol residues;
(b) a residue $R_1CO$, where $R_1$ is a linear or branched $C_{11}$-$C_{29}$ aliphatic radical;
(c) a residue

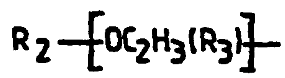

where:

- $R_2$ may have the meaning (a) or (b) given for $R_0$;
- $-OC_2H_3(R_3)-$
is represented by the following structures, taken as a mixture or separately:

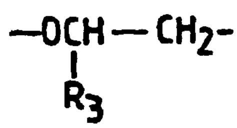 and 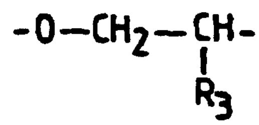

where $R_3$ has the meaning (a) given for $R_0$;

(2) linear or branched polyglycerol ethers comprising two fatty chains;
(3) fatty chain diols;
(4) fatty alcohols which are oxyethylenated or otherwise, sterols and phytosterols, oxyethylenated or otherwise;
(5) ethers and esters of polyols, oxyethylenated or otherwise, it being possible for the linkage of the ethylene oxides to be linear or cyclic;
(6) glycolipids of natural or synthetic origin, ethers and esters of mono- or polysaccharides and in particular the ethers and esters of glucose;
(7) the hydroxyamides represented by the formula:

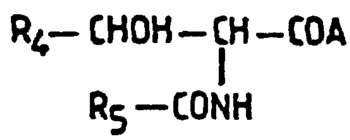 (V)

in which formula (V):

- $R_4$ denotes a $C_7$-$C_{21}$ alkyl or alkenyl radical;
  - $R_5$ denotes a saturated or unsaturated $C_7$-$C_{31}$ hydrocarbon radical;
  - COA denotes a group chosen from the following two groups:
    - a residue

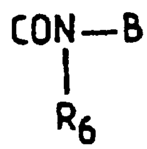

where:

- B is an alkyl radical derived from mono- or polyhydroxylated primary or secondary amines; and
- $R_6$ denotes a hydrogen atom or a methyl, ethyl or hydroxyethyl radical; and
- a residue -COOZ, where Z represents the residue of a $C_3$-$C_7$ polyol.

(8) natural or synthetic ceramides;
(9) dihydroxyalkylamines, fatty amines which are oxyethylenated;
(10) the derivatives of glycerol corresponding to the formula:

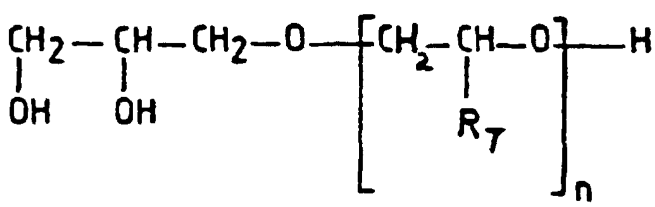 (VI)

in which formula (VI) $R_7$ represents a linear $C_{14}$ to $C_{18}$ alkyl radical or a $-CH_2A$ group in which A is $-OR_{14}$, $R_{14}$ representing a linear $C_{10}$-$C_{18}$ and, preferably, $C_{16}$, alkyl radical, and n represents a mean statistical value greater than 1 and at most equal to 3 and, in addition, when $R_7$ = $-CH_2A$, n may also represent an actual value (not statistical) equal to 2.

(B) the ionic amphiphilic lipids defined below:

(1) the anionic amphiphilic lipids chosen from the group formed by:

- natural phospholipids, phospholipids modified chemically or enzymatically, and synthetic phospholipids;
- anionic compounds represented by the formula:

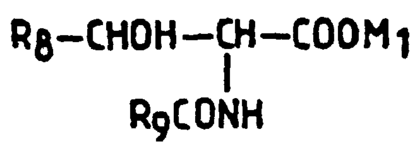 (VII)

in which formula (VII):

- $R_8$ represents a $C_7$-$C_{21}$ alkyl or alkenyl radical
- $R_9$ represents a saturated or unsaturated $C_7$-$C_{31}$ hydrocarbon radical, and
- $M_1$ represents H, Na, K, $NH_4$ or a substituted ammonium ion derived from an amine,

(2) the anionic compounds chosen from the group formed by:
the phosphoric esters of fatty alcohols; heptylnonylbenzenesulphonic acid; cholesterol acid sulphate and its alkali metal salts and cholesterol acid phosphate and its alkali metal salts;
lysolecithins; alkyl sulphates; gangliosides;
(3) the cationic amphiphilic lipids chosen from the group formed by:

- the quaternary ammonium-derived cationic compounds corresponding to the formula:

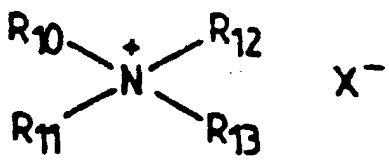

(VIII)

with $R_{10}$ and $R_{11}$, which are identical or different, representing $C_{12}$-$C_{20}$ alkyl radicals and $R_{12}$ and $R_{13}$, which are identical or different, $C_1$-$C_4$ alkyl radicals;
- long-chain amines and their quaternary ammonium derivatives, long-chain amino alcohol esters and their salts and quaternary ammonium derivatives;
- the prepolymerized amphiphilic lipids obtained in particular from the polymerizable lipids or from the reaction of an anionic lipid with a polymerizable cationic compound.

**13.** Composition according to one of Claims 7 to 12, characterized in that the aqueous dispersing phase consists of water or a mixture of water and at least one $C_1$-$C_7$ alcohol and/or one $C_1$-$C_5$ alkyl polyol.

**14.** Composition according to one of Claims 7 to 13, characterized in that a fatty phase is combined with the aqueous dispersing phase.

**15.** Composition according to Claim 14, characterized in that the fatty phase is in the form of a water-in-oil or oil-in-water type emulsion, the aqueous phase of the emulsion consisting of the aqueous dispersing phase of the vesicles.

**16.** Composition according to either of Claims 14 and 15, characterized in that the fatty phase consists of at least one compound chosen from the group consisting of oils and fatty substances of animal or plant origin, natural or synthetic essential oils, waxes, hydrocarbons, halogenated hydrocarbons, silicones, esters of an inorganic acid and an alcohol, and ethers and polyethers.

**17.** Composition according to one of Claims 14 to 16, characterized in that the fatty phase represents from 5 to 50% by weight relative to the total weight of the composition.

**18.** Composition according to one of Claims 7 to 17, characterized in that it contains at least one cosmetic and/or pharmaceutical active agent.

**19.** Composition according to one of Claims 7 to 18, characterized in that it contains at least one formulation adjuvant.

**Patentansprüche**

**1.** Verfahren zur Stabilisierung von Vesikeln, die von einer mindestens ein amphiphiles ionisches und/oder nichtionisches Lipid enthaltenden Lipidphase-Membran gebildet sind, in der eine wässrige Phase verkapselt ist, in Form von Dispersion in einer wässrigen Phase durch Einführung mindestens eines Stabilisierungsmittels in die wässrige Dispersionsphase, dadurch gekennzeichnet, dass das (die) Stabilisierungsmittel aus der Gruppe ausgewählt sind, die aus Glycerinalginaten, Propylenglykolalginaten, Gelangummi und Welangummi besteht.

**2.** Verfahren nach Anspruch 1, dadurch gekennzeichnet, dass das Stabilisierungsmittel ein zu mehr als 60% mit Propylenglykol oder Glycerin verestertes Alginat ist.

**3.** Verfahren nach einem der Ansprüche 1 oder 2, dadurch gekennzeichnet, dass das (die) Stabilisierungsmittel in einer Menge von 0,1 bis 20 Gew.-%, bezogen auf das Gewicht der vesikulären Lipidphase, verwendet wird (werden).

**4.** Verfahren nach Anspruch 3, dadurch gekennzeichnet, dass das (die) Stabilisierungsmittel in einer Menge von 0,2 bis 10 Gew.-%, bezogen auf das Gewicht der vesikulären Lipidphase, verwendet wird (werden).

**5.** Verfahren nach einem der Ansprüche 1 bis 4, dadurch gekennzeichnet, dass die Zusammensetzung bis zu 20 Gew.-% Tensid(e), bezogen auf das Gesamtgewicht der Zusammensetzung, enthält.

**6.** Verfahren nach einem der Ansprüche 1 bis 5, dadurch gekennzeichnet, dass der wässrigen Dispersionsphase eine Fettphase zugeordnet ist, die bis zu 50 Gew.-% der wässrigen Dispersionsphase darstellt.

**7.** Zusammensetzung zur topischen Anwendung, die erstens eine Dispersion von Vesikeln, die von einer mindestens ein amphiphiles ionisches und/oder nichtionisches Lipid enthaltenden Lipidphase-Membran gebildet sind, in der eine wässrige Phase verkapselt ist, in wässriger Phase und zweitens mindestens ein diese Vesikel stabilisierendes Mittel enthält, dadurch gekennzeichnet, dass das (die) Stabilisierungsmittel aus der Gruppe ausgewählt ist (sind), die aus Glycerinalginaten, Propylenglykolalginaten, Gelangummi und Welangummi besteht.

**8.** Zusammensetzung nach Anspruch 7, dadurch gekennzeichnet, dass das (die) Stabilisierungsmittel 0,1 bis 20 Gew.-%, bezogen auf das Gewicht der vesikulären Lipidphase, darstellt (darstellen).

**9.** Zusammensetzung nach Anspruch 8, dadurch gekennzeichnet, dass das (die) Stabilisierungsmittel 0,2 bis 10 Gew.-%, bezogen auf das Gewicht der vesikulären Lipidphase, darstellt (darstellen).

**10.** Zusammensetzung nach einem der Ansprüche 7 bis 9, dadurch gekennzeichnet, dass die Zusammensetzung 1 bis 20 Gew.-% Tensid(e), bezogen auf das Gesamtgewicht der Zusammensetzung, enthält.

**11.** Zusammensetzung nach Anspruch 10, dadurch gekennzeichnet, dass das Tensid aus der Gruppe ausgewählt ist, die aus Natriumalkylsulfaten, Polyolestern und oxyethylenierten Polyolestern, polyoxyethylenierten Alkoholen, polypropylenierten Alkoholen und Polyoxyethylen/Polyoxypropylen-Copolymeren besteht.

**12.** Zusammensetzung nach einem der Ansprüche 7 bis 11, dadurch gekennzeichnet, dass die vesikuläre Lipidphase mindestens ein Lipid enthält, das aus der aus folgenden Stoffen bestehenden Gruppe ausgewählt ist:

A) folgende nichtionische Lipide:

1) verzweigte oder unverzweigte Glycerinderivate der Formel (IV)

$$R_0O-[C_3H_5(OH)O]_{\bar{n}}-H \quad (IV)$$

in welcher:

- $-C_3H_5(OH)O-$ durch die folgenden Strukturen in Mischung oder einzeln dargestellt wird:

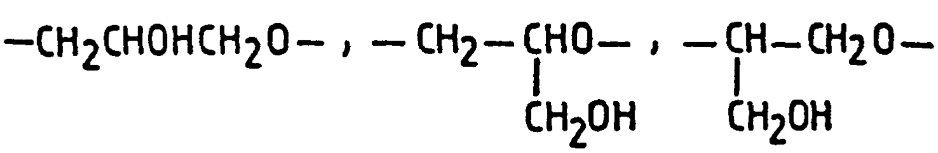

- $\bar{n}$ ein mittlerer statistischer Wert zwischen 1 und 6 ist oder n = 1 oder 2, wobei $-C_3H_5(OH)O-$ durch die Struktur $-CH_2CHOH-CH_2O-$ dargestellt wird;
- $R_0$ folgendes darstellt:

  a) eine gesättigte oder ungesättigte verzweigte oder unverzweigte aliphatische Kette, die 12 bis 30 Kohlenstoffatome enthält; oder Kohlenwasserstoffreste der Lanolinalkohole; oder langkettige alpha-Diol-Reste;
  b) ein Rest $R_1CO$, in dem $R_1$ ein verzweigter oder unverzweigter aliphatischer Rest mit $C_{11}$-$C_{29}$ ist;
  c) ein Rest

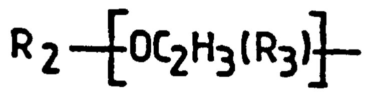

worin:

. $R_2$ die Bedeutung a) oder b) von $R_0$ haben kann;
. $-OC_2H_3(R_3)-$
durch die folgenden Strukturen in Mischung oder einzeln dargestellt ist:

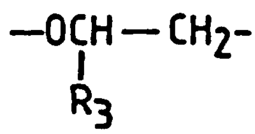 und 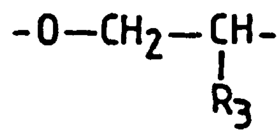

worin $R_3$ die Bedeutung a) von $R_0$ hat;

2) lineare oder verzweigte Polyglycerinether mit zwei Fettsäureketten;
3) Diole mit Fettsäurekette;
4) Fettalkohole, oxyethyleniert oder nicht, Sterine und Phytosterine, oxyethyleniert oder nicht;
5) Polyolether oder -ester, oxyethyleniert oder nicht, wobei die Verkettung der Ethylenoxide linear oder zyklisch sein kann;
6) Glykolipide natürlichen oder synthetischen Ursprungs, Mono- oder Polysaccharidether oder -ester und insbesondere Glucoseether und -ester;
7) Hydroxyamide der Formel (V)

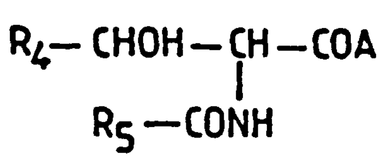 (V)

in der

- $R_4$ einen Alkyl- oder Alkenylrest mit $C_7$-$C_{21}$ bezeichnet;
- $R_5$ einen gesättigten oder ungesättigten Kohlenwasserstoffrest mit $C_7$-$C_{31}$ bezeichnet;
- COA eine aus den beiden folgenden Gruppen ausgewählte Gruppe bezeichnet:

  - einen Rest

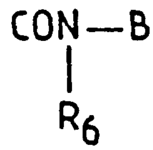

in dem:

- . B ein von primären oder sekundären mono- oder polyhydroxylierten Aminen abgeleiteter Alkoylrest ist und
- . $R_6$ ein Wasserstoffatom oder einen Methyl-, Ethyl- oder Hydroxyethylrest bezeichnet, und

- • einen Rest -COOZ, in dem Z den Rest eines Polyols mit $C_3$-$C_7$ darstellt;

8) natürliche oder synthetische Ceramide;
9) Dihydroxyalkylamine, oxyethylenierte Fettamine;
10) Derivate von Glycerin, die der Formel (VI)

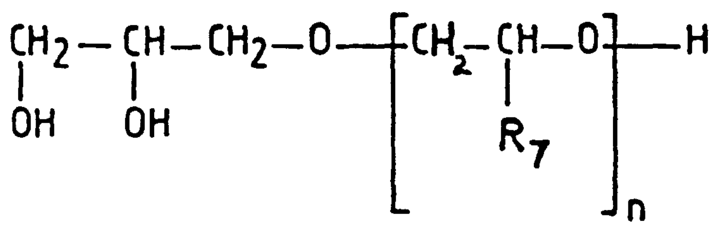

(VI)

entsprechen, in der $R_7$ einen linearen Alkylrest mit $C_{14}$ bis $C_{18}$ oder eine Gruppe $-CH_2A$ darstellt, in der A für $-OR_{14}$ steht, wobei $R_{14}$ einen linearen Alkylrest mit $C_{10}$-$C_{18}$ und vorzugsweise $C_{16}$ darstellt, und n einen mittleren statistischen Wert größer als 1 und höchstens gleich 3 darstellt, wobei, wenn $R_7 = -CH_2A$, n außerdem auch einen realen (nicht statistischen) Wert gleich 2 darstellen kann;

B) die folgenden amphiphilen ionischen Lipide:

1) anionische amphiphile Lipide, die aus der aus den folgenden Stoffen bestehenden Gruppe ausgewählt sind:

- • natürliche Phospholipide, chemisch oder enzymatisch modifizierte Phospholipide und synthetische Phospholipide;
- • anionische Verbindungen der Formel (VII)

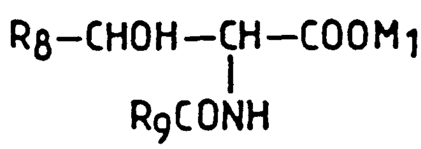

(VII)

in welcher:

- . $R_8$ einen Alkyl- oder Alkenylrest mit $C_7$-$C_{21}$ darstellt,
- . $R_9$ einen gesättigten oder ungesättigten Kohlenwasserstoffrest mit $C_7$-$C_{31}$ darstellt und
- . $M_1$ für H, Na, K, $NH_4$ oder ein von einem Amin abgeleitetes substituiertes Ammoniumion steht,

2) anionische Verbindungen, die aus der aus den folgenden Stoffen bestehenden Gruppe ausgewählt sind: Fettalkohol-Phosphorester; Heptylnonylbenzolsulfonsäure; saures Cholesterinsulfat und seine alkalischen Salze und saures Cholesterinphosphat und seine alkalischen Salze; Lysolecithine; Alkylsulfate; Ganglioside;
3) kationische amphiphile Lipide, die aus der aus den folgenden Stoffen bestehenden Gruppe ausgewählt sind:

- von quartärem Ammonium abgeleitete kationische Verbindungen der Formel

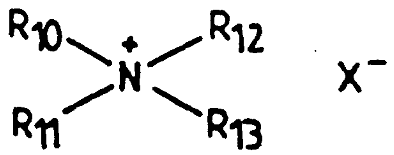

(VIII)

worin $R_{10}$ und $R_{11}$ gleich oder verschieden sind und Alkylreste mit $C_{12}$-$C_{20}$ darstellen und $R_{12}$ und $R_{13}$ gleich oder verschieden sind und Alkylreste mit $C_1$-$C_4$ darstellen;
- Amine langer Kette und ihre quartären Ammoniumderivate, Aminoalkoholester langer Kette und ihre Salze und quartäre Ammoniumderivate;
- polymerisierte amphiphile Lipide, die aus polymerisierbaren Lipiden oder durch Reaktion eines anionischen Lipids und einer polymerisierbaren kationischen Verbindung erhalten werden.

**13.** Zusammensetzung nach einem der Ansprüche 7 bis 12, dadurch gekennzeichnet, dass die wässrige Dispersionsphase aus Wasser oder aus einer Mischung von Wasser und mindestens einem $C_1$-$C_7$-Alkohol und/oder einem $C_1$-$C_5$-Alkylpolyol besteht.

**14.** Zusammensetzung nach einem der Ansprüche 7 bis 13, dadurch gekennzeichnet, dass der wässrigen Dispersionsphase eine Fettphase zugeordnet ist.

**15.** Zusammensetzung nach Anspruch 14, dadurch gekennzeichnet, dass die Fettphase in Form einer Wasser-in-Öl- oder Öl-in-Wasser-Emulsion vorliegt, wobei die wässrige Phase der Emulsion aus der wässrigen Vesikeldispersionsphase besteht.

**16.** Zusammensetzung nach einem der Ansprüche 14 oder 15, dadurch gekennzeichnet, dass die Fettphase aus mindestens einer Verbindung besteht, die aus der aus folgenden Stoffen bestehenden Gruppe gewählt ist: Öle und Fette tierischen oder pflanzlichen Ursprungs, natürliche oder synthetische essentielle Öle, Wachse, Kohlenwasserstoffe, halogenierte Kohlenstoffe, Silicone, Ester aus mineralischer Säure und einem Alkohol und Ether und Polyether.

**17.** Zusammensetzung nach einem der Ansprüche 14 bis 16, dadurch gekennzeichnet, dass die Fettphase 5 bis 50 Gew.-%, bezogen auf das Gesamtgewicht der Zusammensetzung, darstellt.

**18.** Zusammensetzung nach einem der Ansprüche 7 bis 17, dadurch gekennzeichnet, dass sie mindestens einen kosmetischen und/oder pharmazeutischen Wirkstoff enthält.

**19.** Zusammensetzung nach einem der Ansprüche 7 bis 18, dadurch gekennzeichnet, dass sie mindestens einen Rezepturhilfsstoff enthält.